(19) Europäisches Patentamt
European Patent Office
Office européen des brevets

(11) Publication number: **0 001 290**
**B1**

(12) # EUROPEAN PATENT SPECIFICATION

(45) Date of publication of patent specification: **27.06.84**

(21) Application number: **78100969.1**

(22) Date of filing: **22.09.78**

(51) Int. Cl.³: **C 07 D 275/06,**
C 07 D 279/02,
C 07 D 417/10,
C 07 D 417/12,
C 07 D 417/14,
C 07 D 455/02, C 07 F 7/18
//C09B11/24, C07C43/20,
G03C1/84

(54) **Substituted 2,3-dihydrobenz-(d)-isothiazole-1,1-dioxides and 2,3-dihydronaphtho-(1,8-de)-1,2-thiazine-1,1-dioxides.**

(30) Priority: **23.09.77 US 836010**
**23.09.77 US 836004**
**23.09.77 US 836008**

(43) Date of publication of application:
**04.04.79 Bulletin 79/7**

(45) Publication of the grant of the patent:
**27.06.84 Bulletin 84/26**

(84) Designated Contracting States:
**DE FR GB NL**

(56) References cited:
**DE - A - 2 105 580**
**DE - A - 2 211 183**
**DE - C - 100 779**
**DE - C - 100 780**
**GB - A - 1 363 870**

**JOURNAL OF THE CHEMICAL SOCIETY, Perkin I, 1974, no. 22, RUDOLPH A. ABRAMOVITCH et al. "1.2-Benzisothiazole 1.1-dioxides. Synthesis of 3-alkyl-(or aryl-) 1.2-benzisothiazole 1.1-dioxides and related compounds", pages 2589 to 2594**

(73) Proprietor: **POLAROID CORPORATION**
**549 Technology Square**
**Cambridge, Massachusetts 02139 (US)**

(72) Inventor: **Bloom, Stanley Morton**
**89 Gordon Road**
**Waban Massachusetts (US)**
Inventor: **Borror, Alan Lawrence**
**18 Heritage Drive**
**Lexington Massachusetts (US)**
Inventor: **Foley, James Walter**
**48 Jupiter Road**
**Andover Massachusetts (US)**

(74) Representative: **Splanemann, Rainer (DE) et al,**
**Patentanwälte Dipl.-Ing. Rainer Splanemann,**
**Dipl.-Chem. Bruno Reitzner Tal 13**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

## Description

The present invention is directed to novel substituted 2,3-dihydrobenz-[d]-isothiazole-1,1-dioxides and 2,3-dihydronaphtho-[1,8-de]-1,2-thiazine-1,1-dioxides which are useful as intermediates in the synthesis of certain 3,3-disubstituted-2,3-dihydrobenz[d]isothiazole-1,1-dioxides possessing a carbonyl group in the 2-position.

Such compounds may be selected for use as classical pH-sensitive indicator dyes or as photographic optical filter agents and filter agent precursors depending upon the 2-substituent of the benz[d]-isothiazole ring.

3-Monoaryl-substituted benzisothiazole-1,1-dioxides and their 3-dehydro derivatives are known from DE—A—21 05 580. These compounds are not suitable as indicator dye precursors but are said to be useful as pharmaceuticals and pharmaceutical intermediates.

Abramovitch et al, J. C. S. Perkin I, 1974, No. 22, pages 2589 to 2594 describe the preparation of 3-(p-methoxyphenyl)-2,3-benz[d]isothiazole-1,1-dioxide and disclose the structure for 3,3-di(p-methoxyphenyl)-2,3-dihydrobenz[d]isothiazole-1,1-dioxide. However, they are silent with regard to protecting groups for phenolic compounds. There is no suggestion that the methoxyphenyl substituent is intended to be a hydroxyphenyl substituent protected with a methyl group that would ultimately be removed to give the free hydroxy substituent. Also, there is no disclosure or suggestion of 3,3-bis-substituted-2,3-dihydrobenz[d]isothiazole-1,1-dioxides wherein the bis 3-substituents are phenyl moieties with p-2′-tetrahydropyranyl, p-methoxymethyl or p-dimethyl-t-butylsilyl ether groups.

The novel compounds of the present invention may be represented by the formula

(I)

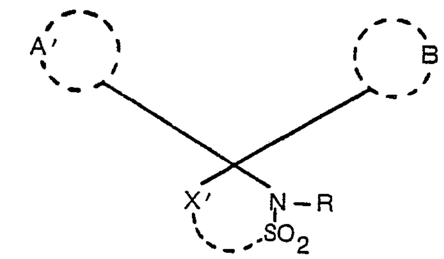

wherein A′ is a 4′-OP-1′-phenyl moiety, unsubstituted or substituted with one or more substituents compatible with organometallic reagents, the same or different, or a 4′-OP-1′-naphthyl moiety, unsubstituted or substituted with one or more substituents compatible with organometallic reagents, the same or different; B is a phenyl moiety, unsubstituted or substituted with one or more substituents compatible with organometallic reagents, the same or different, or a naphthyl moiety, unsubstituted or substituted with one or more substituents compatible with organometallic reagents, the same or different; P is a protecting group selected from 2′-tetrahydropyranyl, methoxymethyl and dimethyl-t-butyl-silyl; X′ represents the atoms necessary to complete a 2,3-dihydrobenz[d]isothiazole-1,1-dioxide moiety or a 2,3-dihydronaphtho[1,8-di]-1,2-thiazine-1,1-dioxide moiety; and R is hydrogen, an alkali metal selected from sodium, potassium and lithium or a carbonyl moiety wherein the —C— is bonded directly to said N atom.

In a preferred embodiment, the novel intermediates of the present invention may be represented by the formula

(II)

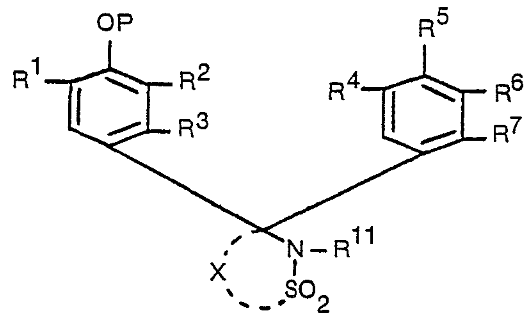

wherein P is a protecting group selected from 2′-tetrahydropyranyl, methoxymethyl and dimethyl-t-butyl-silyl; $R^1$ and $R^2$ are selected from hydrogen, alkyl, alkoxy, chloro and fluoro; $R^3$ is hydrogen, alkyl, alkoxy, or —OP; or $R^2$ and $R^3$ taken together represent the carbon atoms necessary to complete a fused benzene ring; $R^4$ and $R^6$ each are selected from hydrogen, alkyl, alkoxy, chloro and fluoro; $R^5$ is hydrogen, alkyl, alkoxy, —OP′ wherein P′ has the same meaning as P, —N,N-(dialkyl)-amino, —N,N—(W—$R^8$ alkyl)$_2$-amino wherein $R^8$ is halo or —OP″ wherein P″ has the same meaning as P,

—$NHCOCH_3$, piperidino, pyrrolidino, N-methylpiperazino, morpholino, thiomorpholino or tetrahydro-2H,4H-1,3,6-dioxazocino; $R^7$ is hydrogen, alkyl, alkoxy or —$OP^{III}$ wherein $P^{III}$ has the same meaning as P; or $R^6$ and $R^7$ taken together represent the carbon atoms necessary to complete a fused benzene ring; or $R^4$, $R^5$ and $R^6$ taken together represent the atoms necessary to complete a fused [ij]quinolizidine ring; X represents the atoms necessary to complete 2,3-dihydrobenz[d]isothiazole-1,1-dioxide or 2,3-dihydronaphtho[1,8-de]-1,2-thiazine-1,1-dioxide and $R^{11}$ is hydrogen, an alkali metal selected from sodium, potassium and lithium or

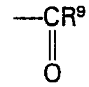

wherein $R^9$ is selected from methyl, methyl substituted with at least one halogen atom selected from chloro, bromo and fluoro, alkoxy having 1 to 4 carbon atoms, phenyl, phenyl substituted in the para position with alkyl having 1 to 4 carbon atoms or —N,N-(dialkyl)-amino, phenyl substituted with at least one electron-withdrawing group, phenoxy, phenoxy substituted with at least one electron-withdrawing group, —$O(CH_2)_2Y$ wherein Y is an electron-withdrawing group and phenyl substituted in the ortho position with —$CH_2R^{10}$ wherein $R^{10}$ is chloro or bromo.

Illustrative compounds are as follows:

(1)

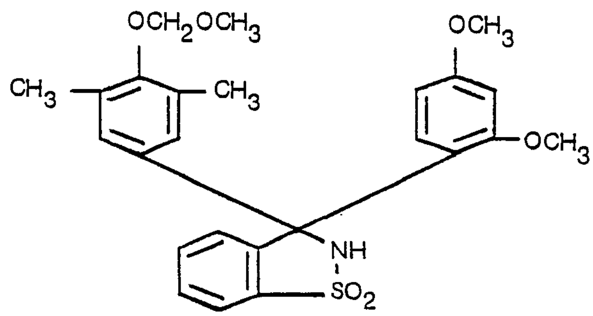

(2)

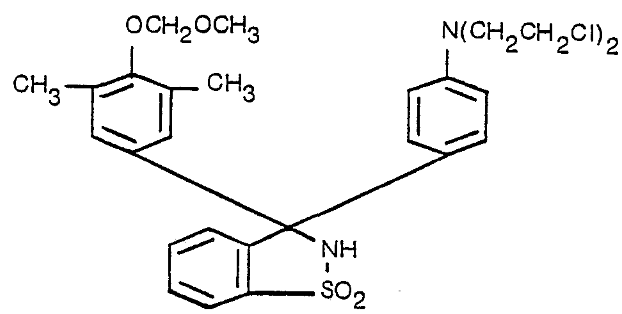

(3)

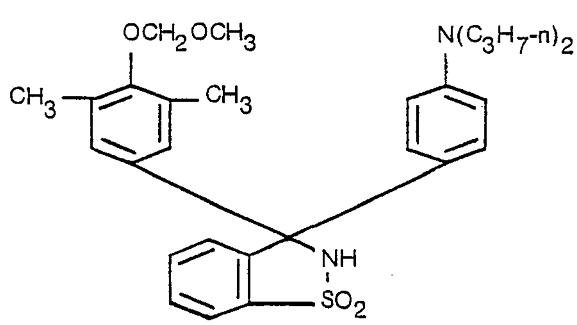

(4)

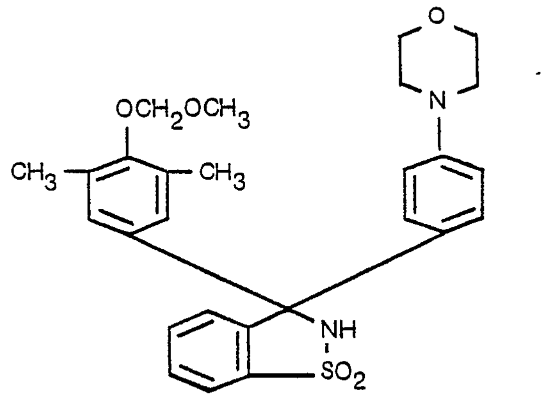

(5)

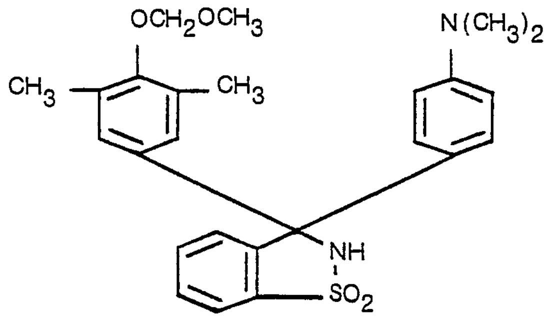

(6)

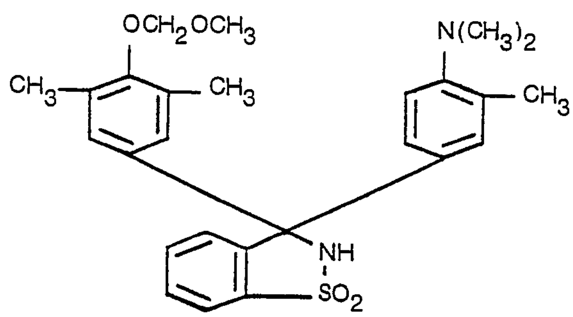

(7)

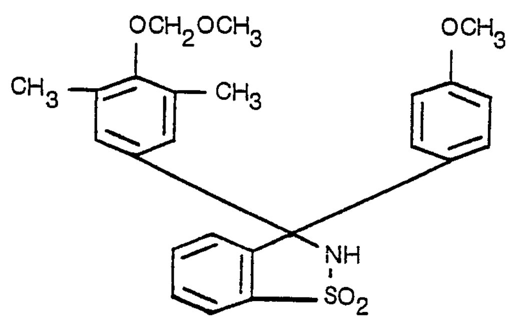

(8)

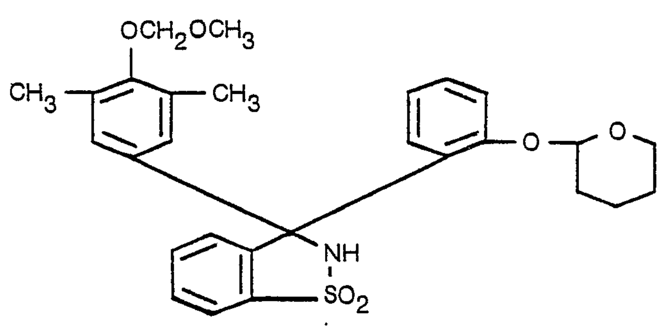

(9)

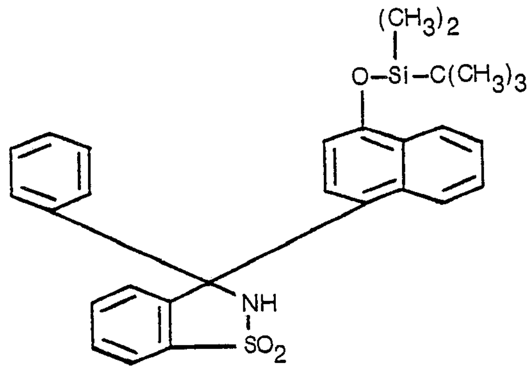

(10)

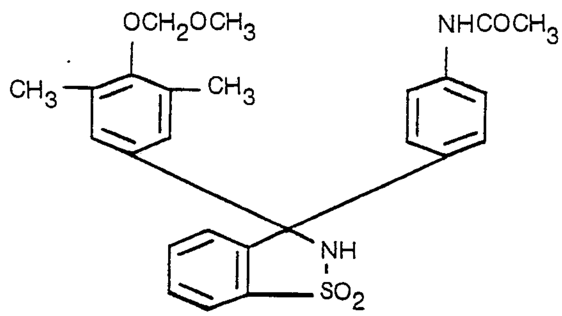

(11)

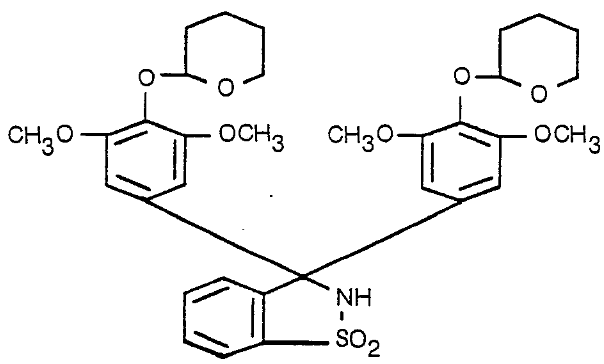

(12)

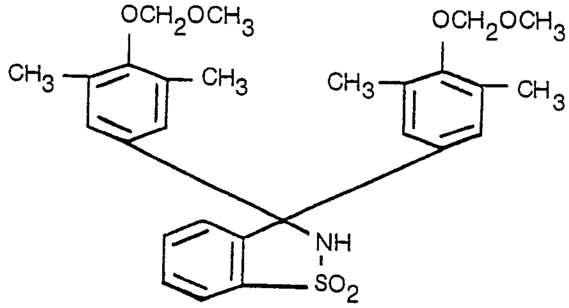

(13)

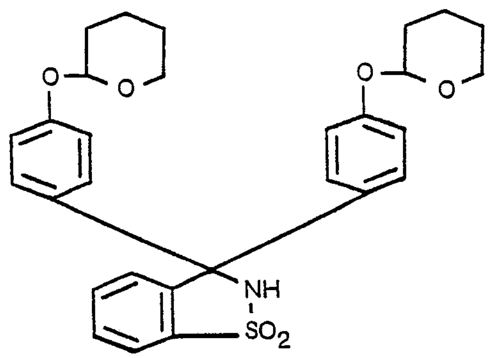

(14)

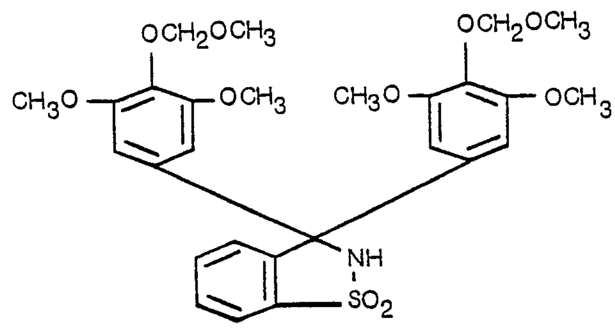

(15)

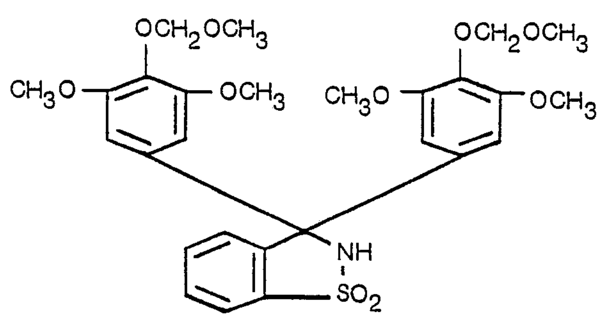

(16)

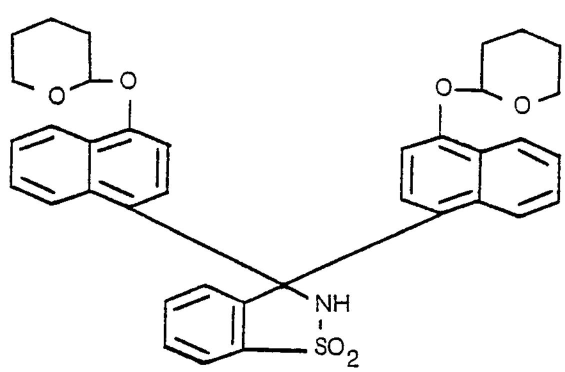

(17)

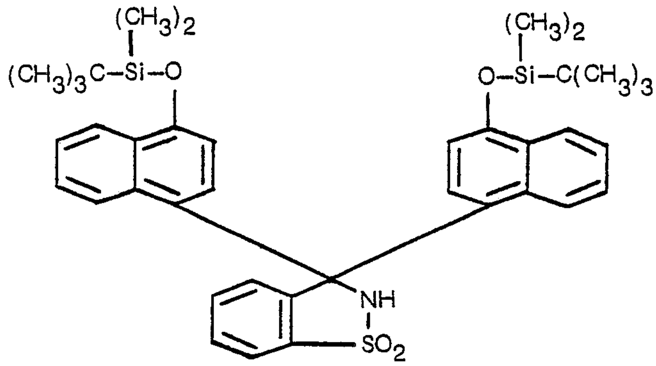

(18)

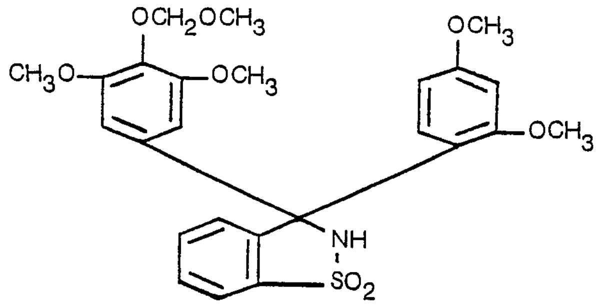

(19)

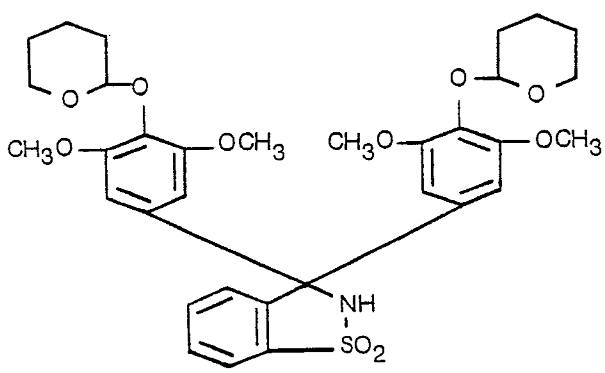

(20)

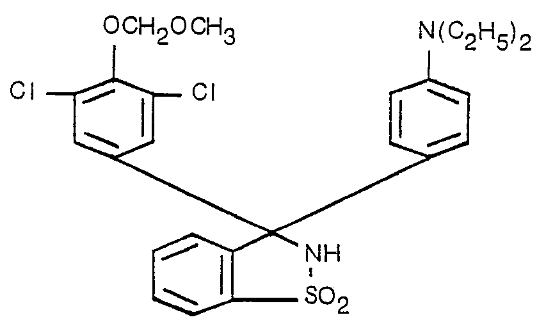

(21)

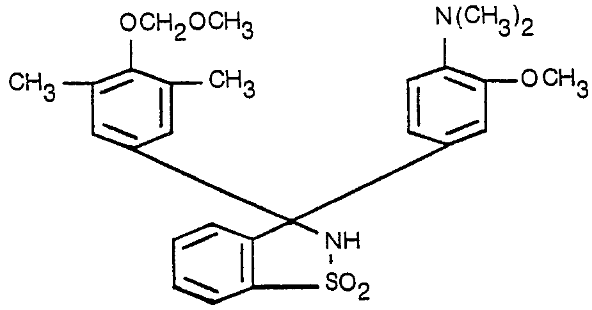

(22)

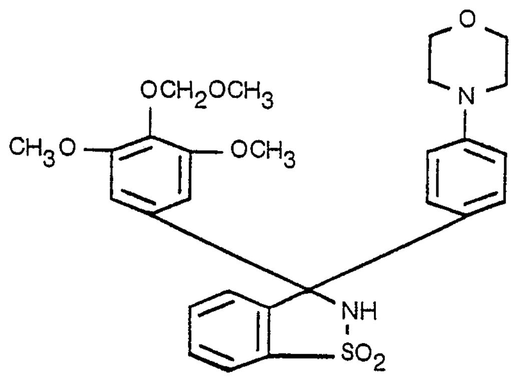

(23)

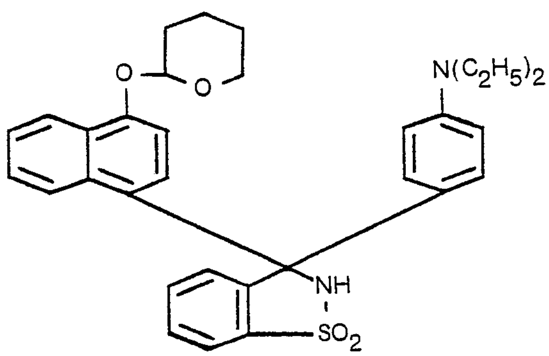

(24)

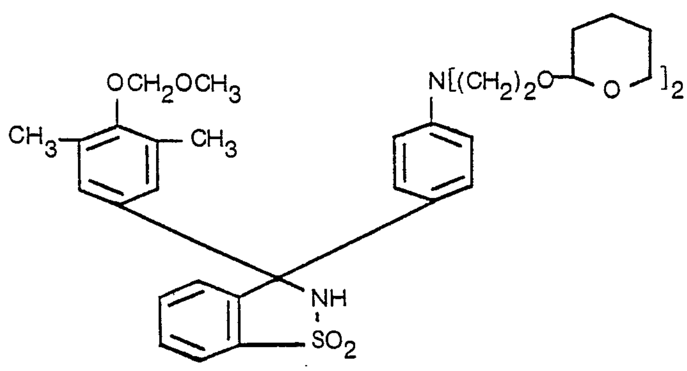

(25)

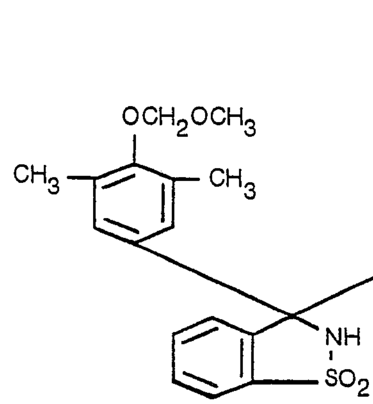

(26)

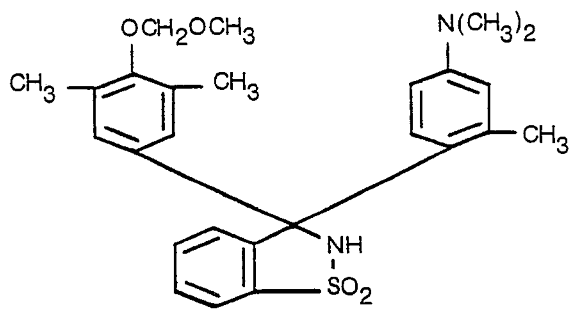

(27)

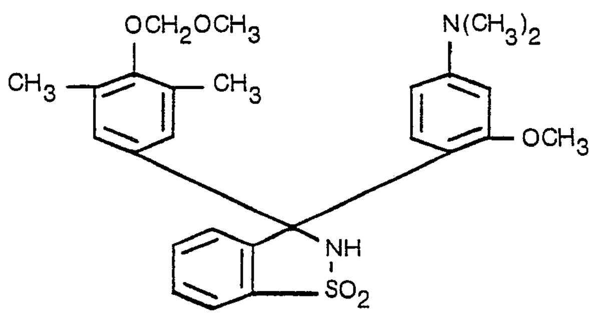

(28)

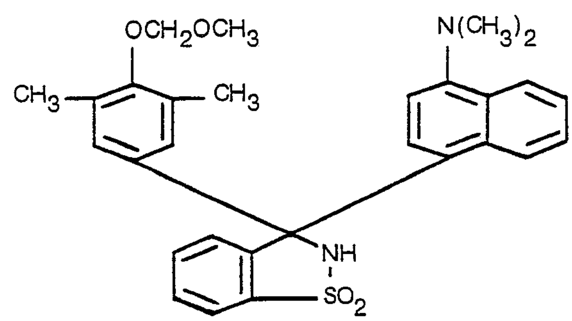

(29)

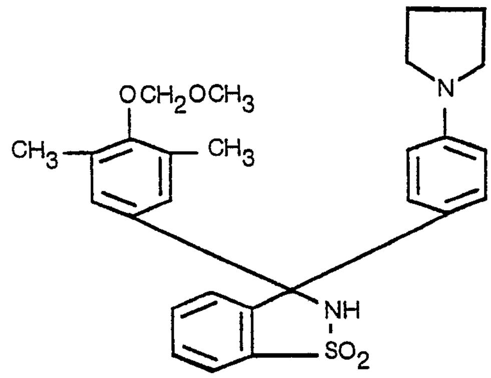

(30)

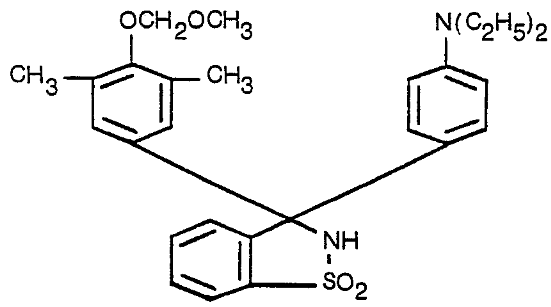

(31)

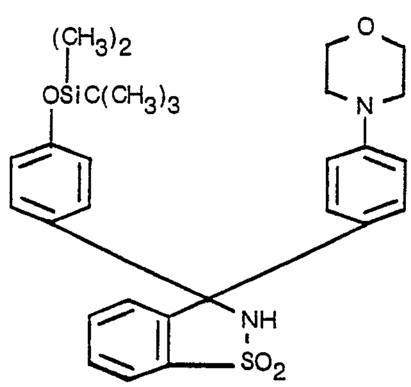

(32)

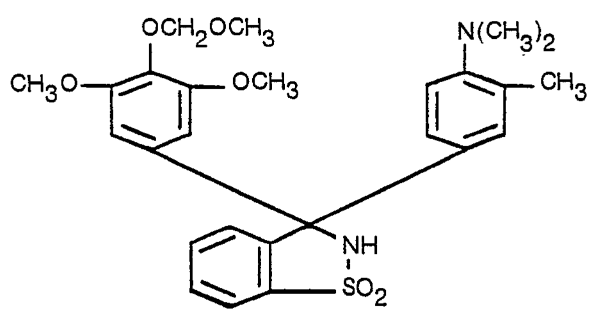

(33)

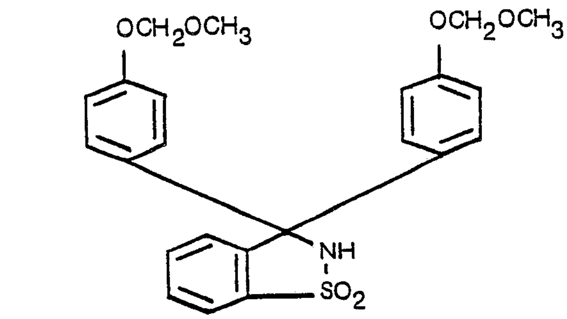

(34)

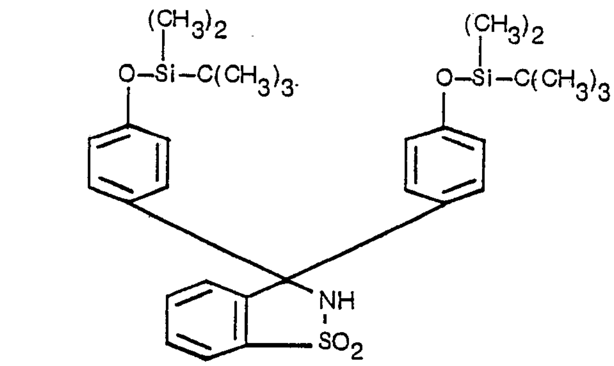

(35)

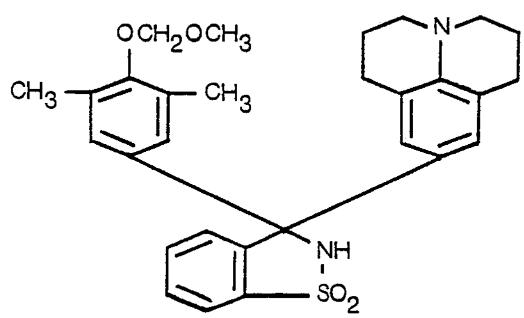

(36)

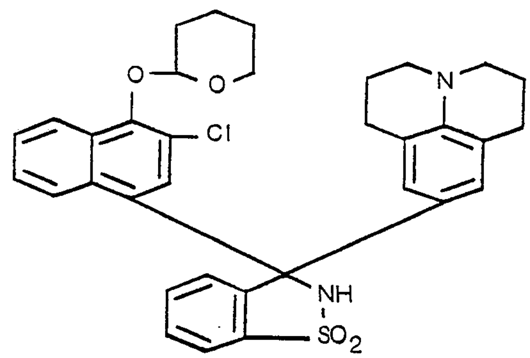

(37)

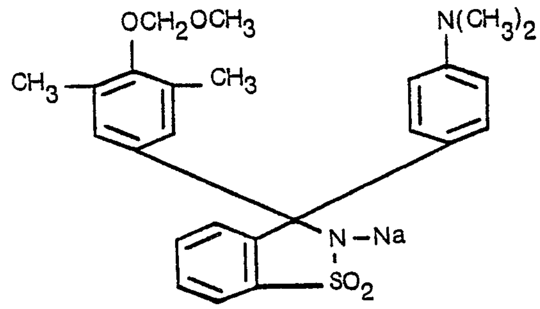

(38)

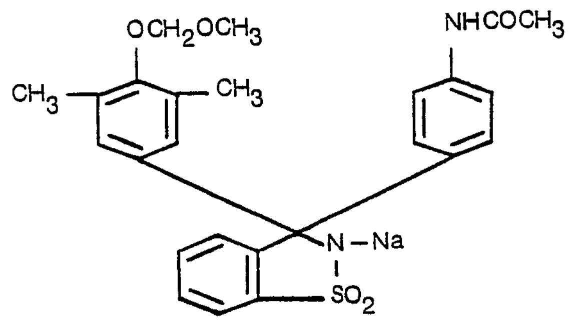

(39)

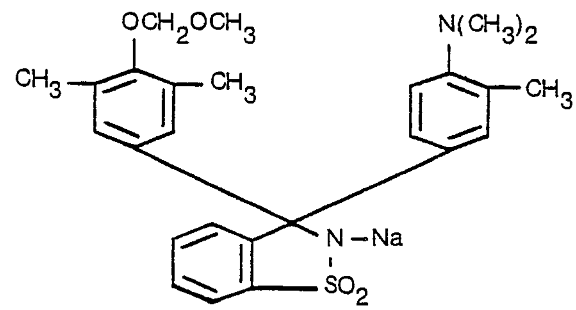

(40)

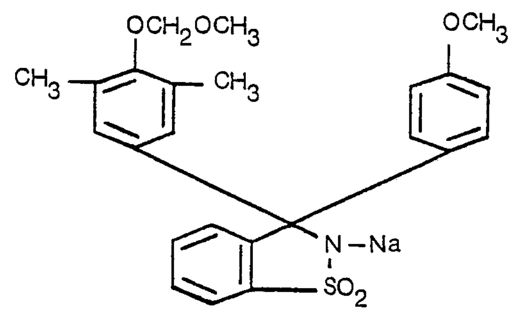

(41)

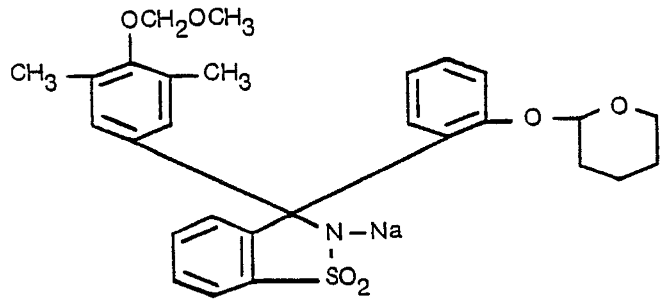

(42)

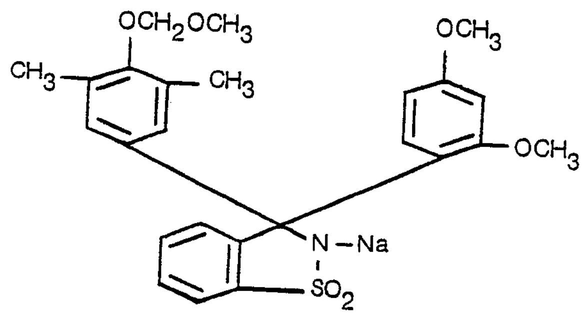

(43)

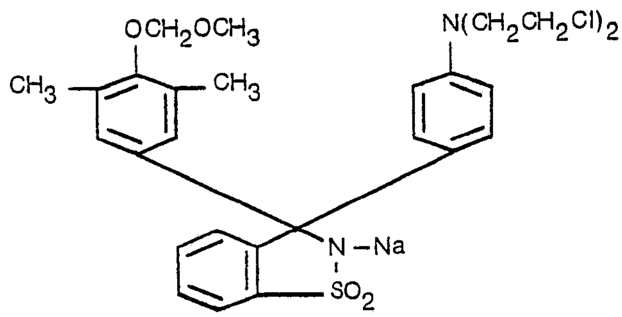

(44)

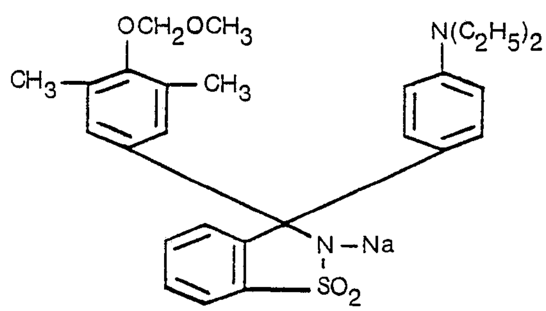

(45)

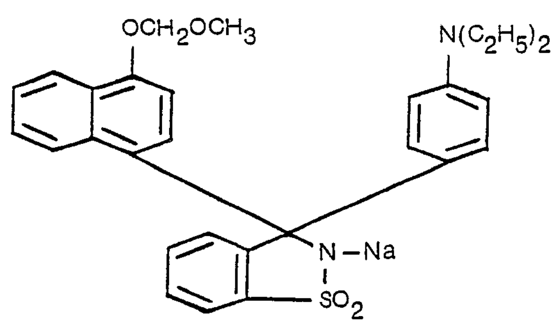

(46)

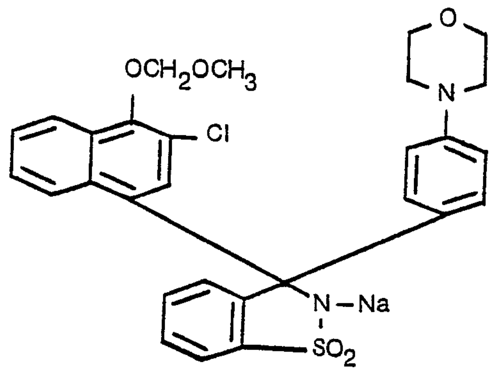

(47)

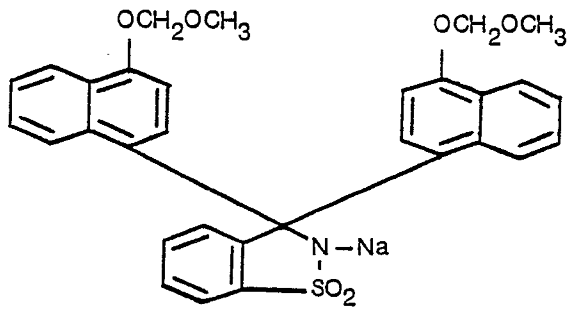

(48)

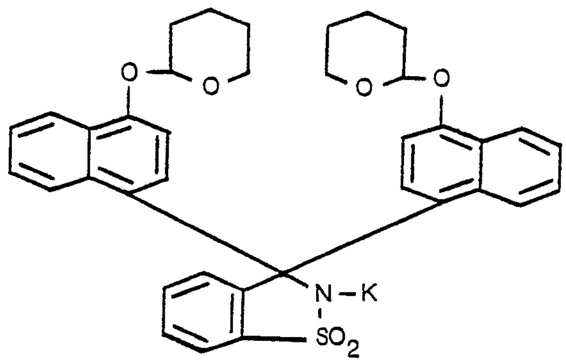

(49)

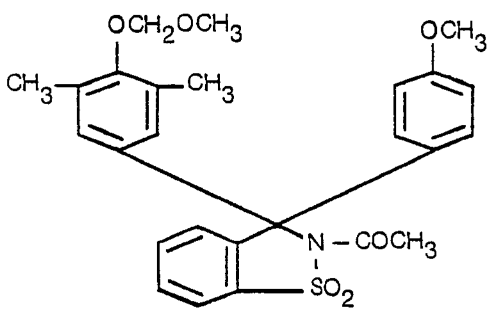

(50)

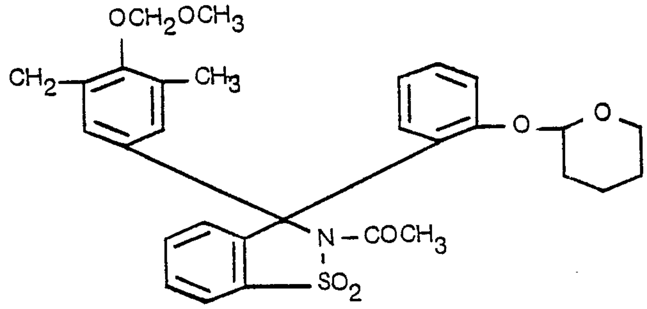

(51)

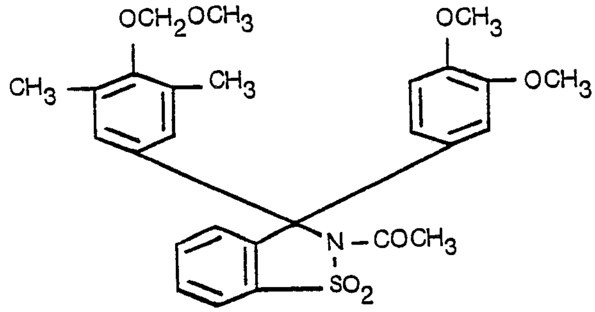

(52)

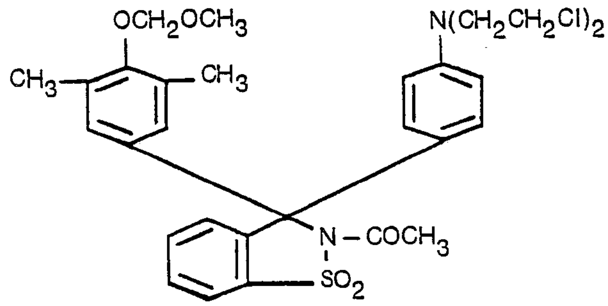

(53)

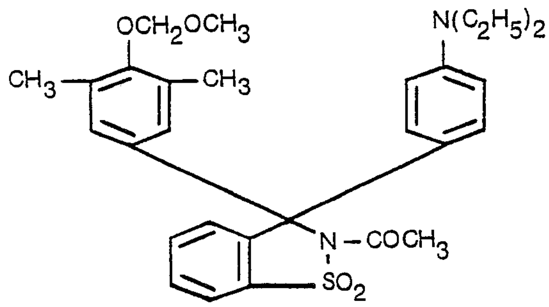

(54)

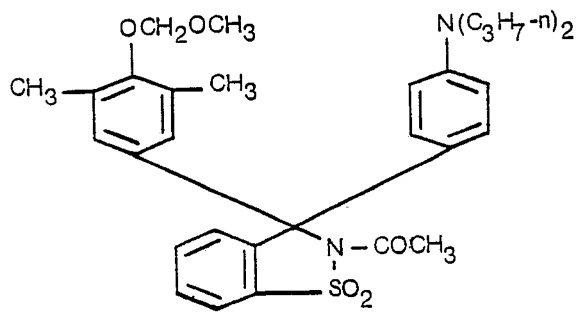

(55)

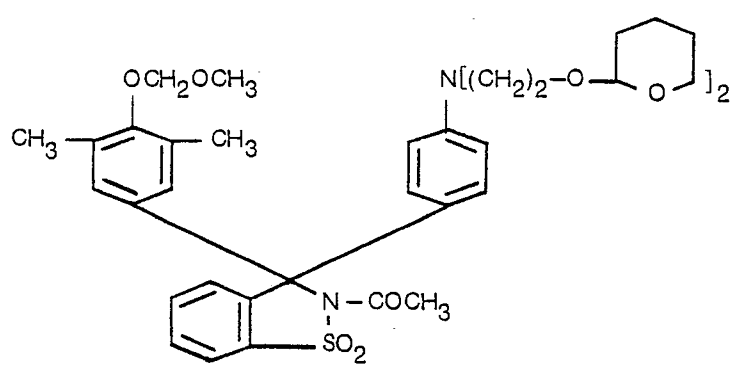

(56)

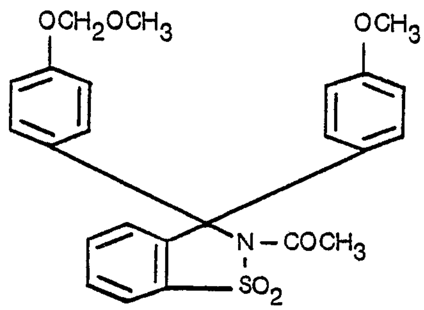

(57)

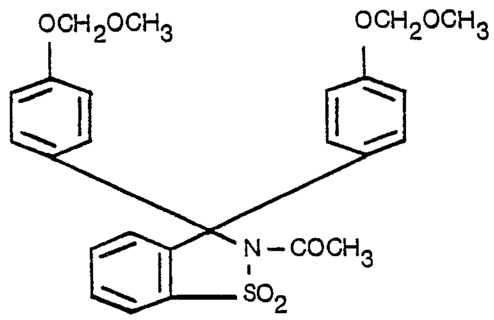

(58)

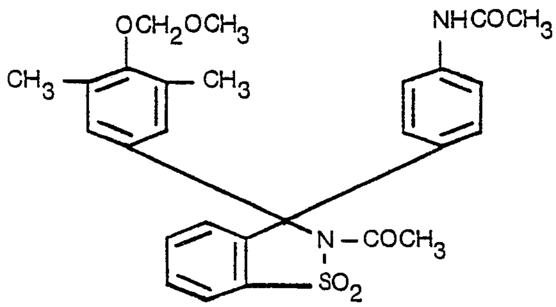

(59)

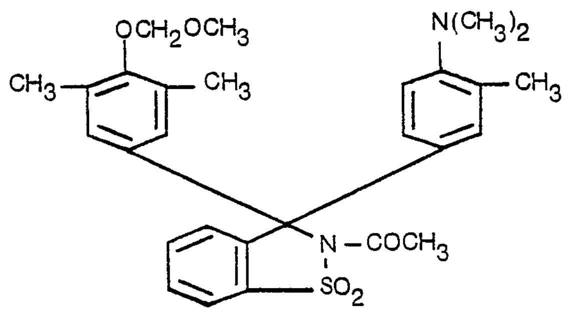

(60)

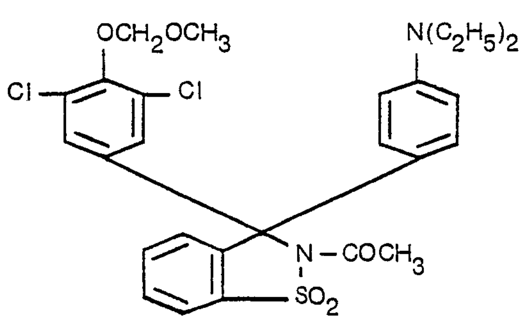

(61)

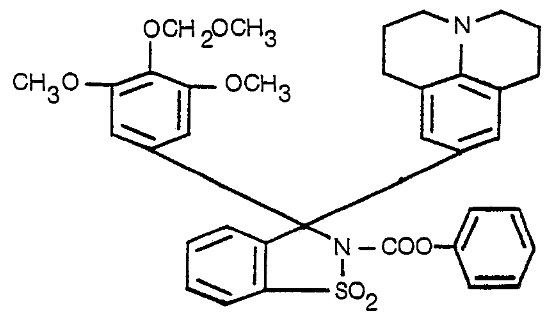

(62)

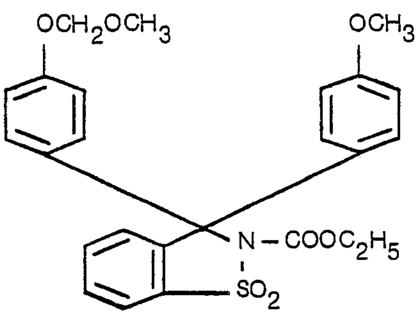

(63)

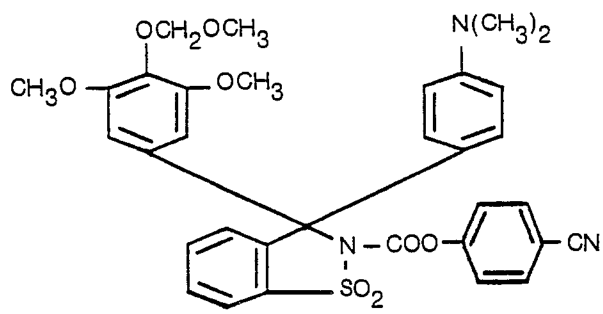

(64)

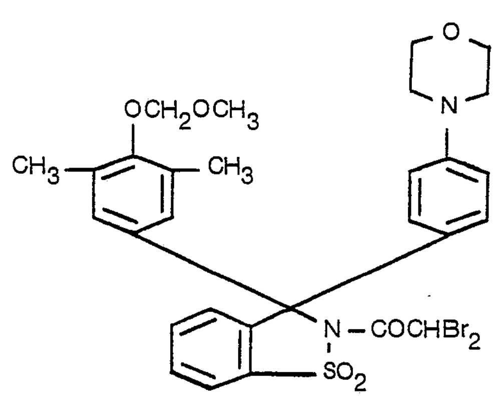

(65)

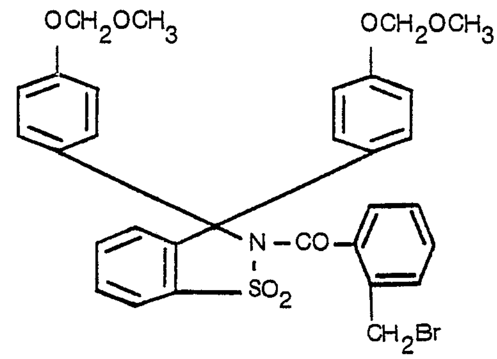

(66)

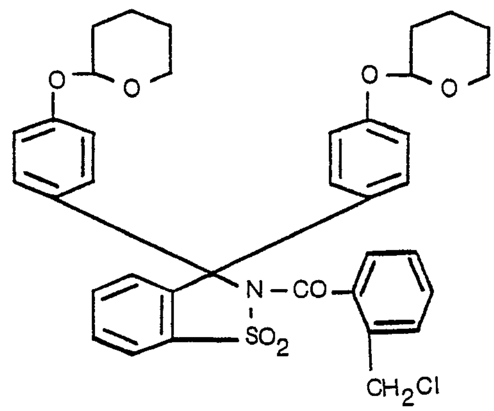

(67)

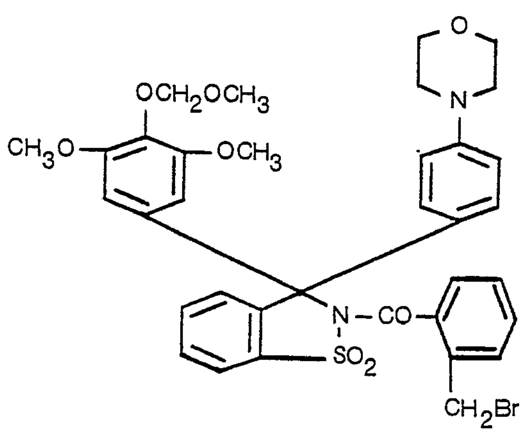

(68)

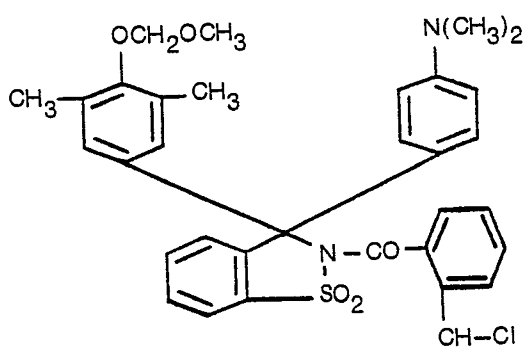

(69)

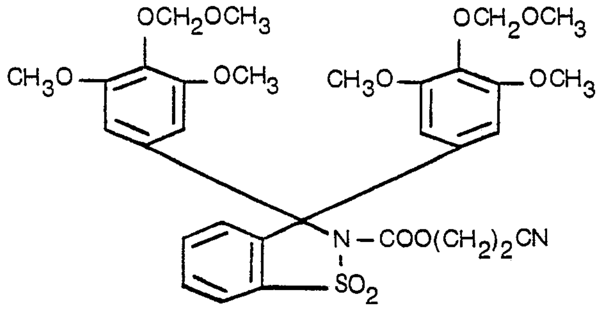

(70)

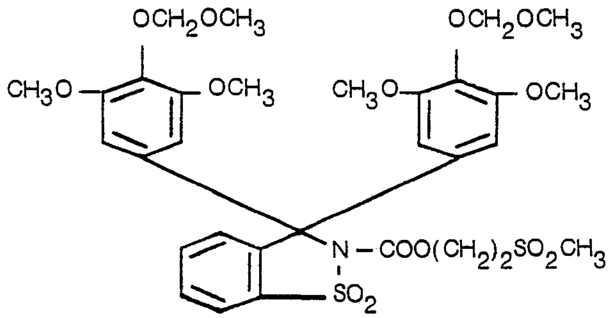

(71)

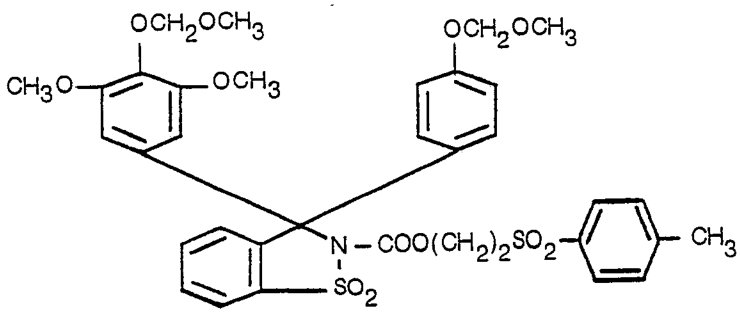

(72)

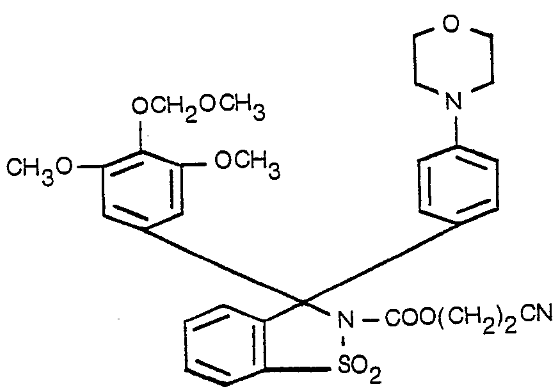

(73)

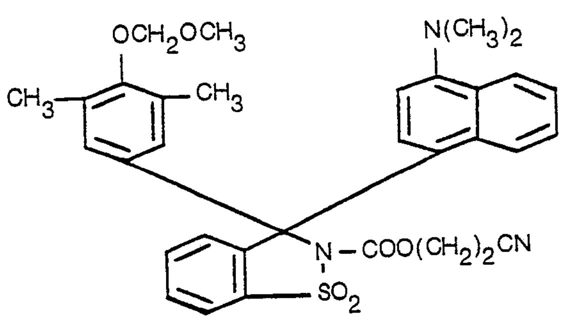

(74)

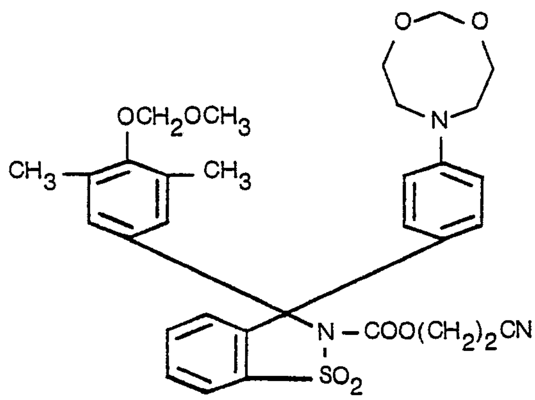

(75)

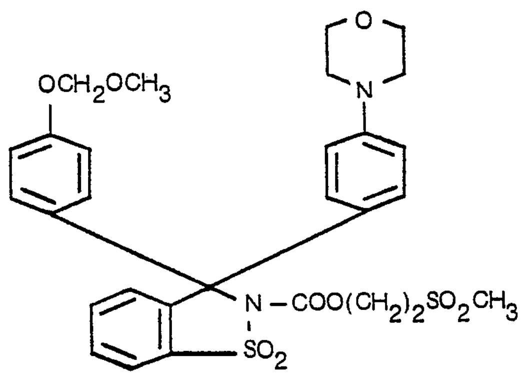

(76)

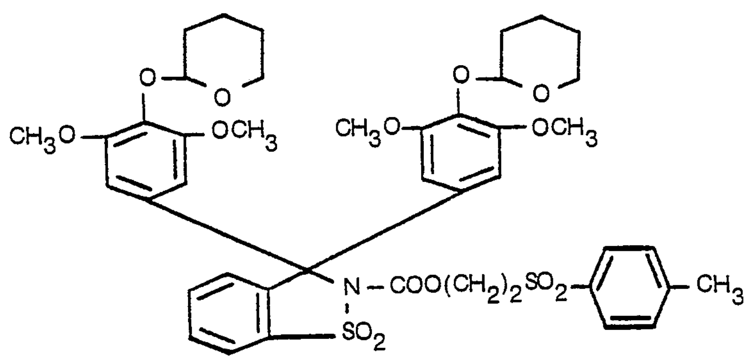

(77)

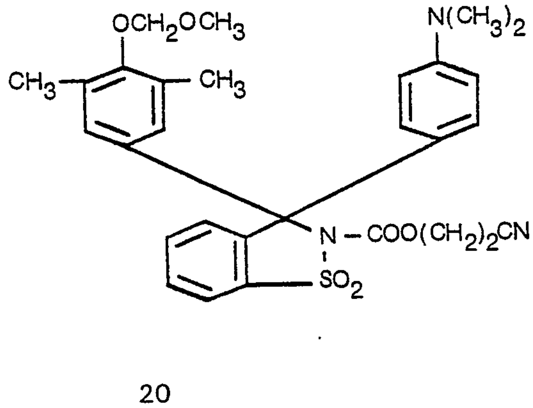

(78)

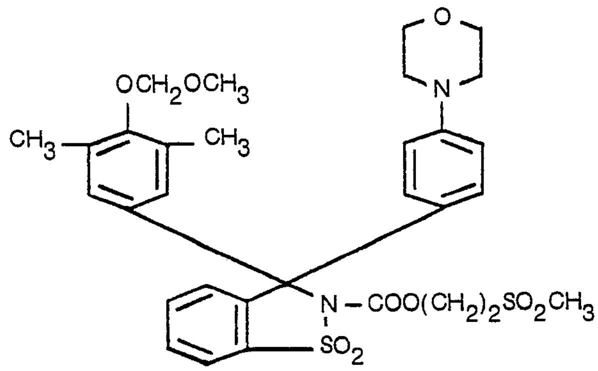

(79)

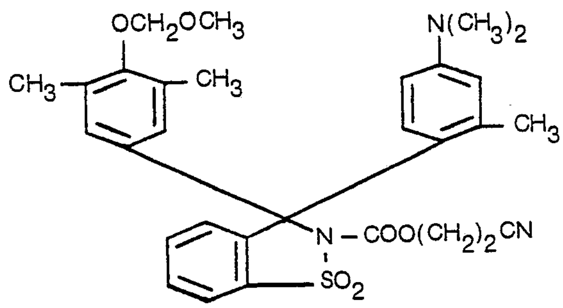

(80)

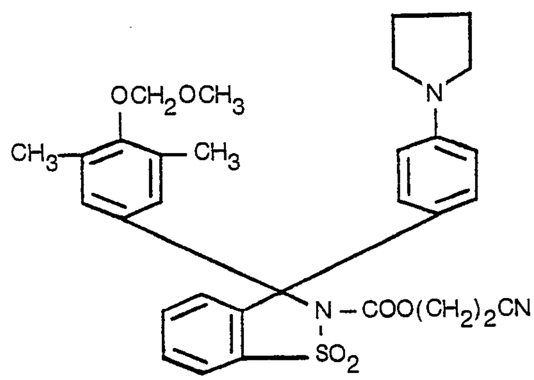

(81)

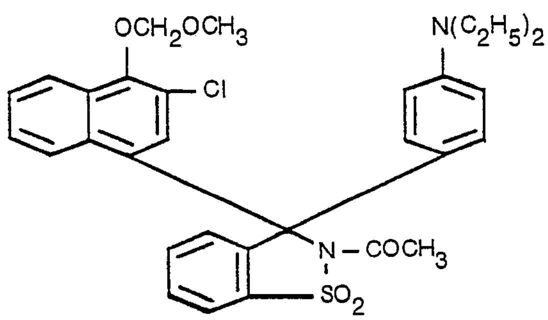

(82)

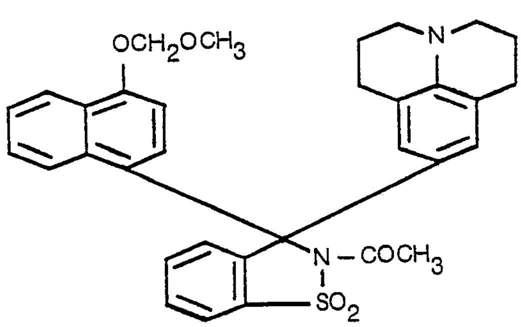

(83)

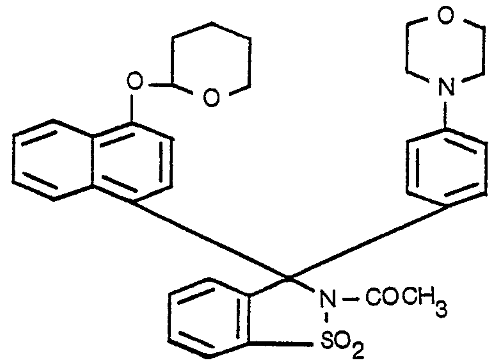

(84)

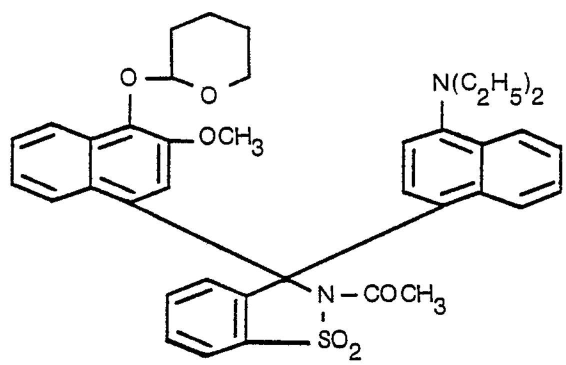

(85)

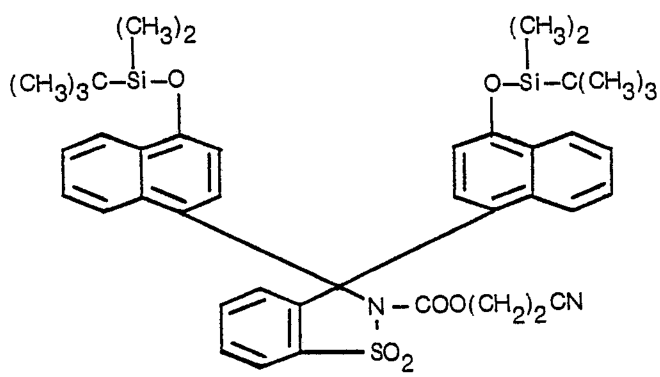

(86)

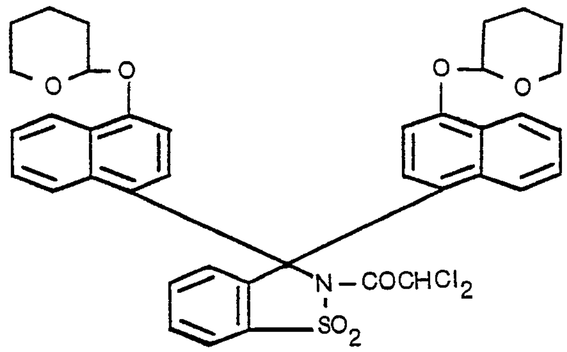

(87)

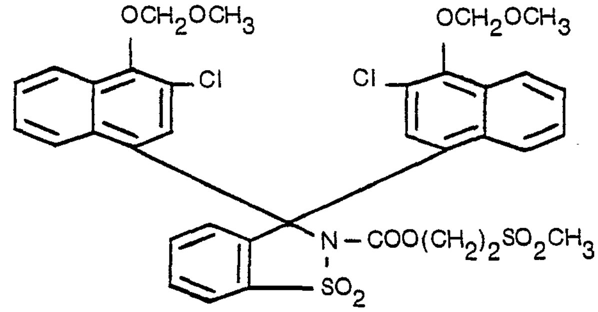

(88)

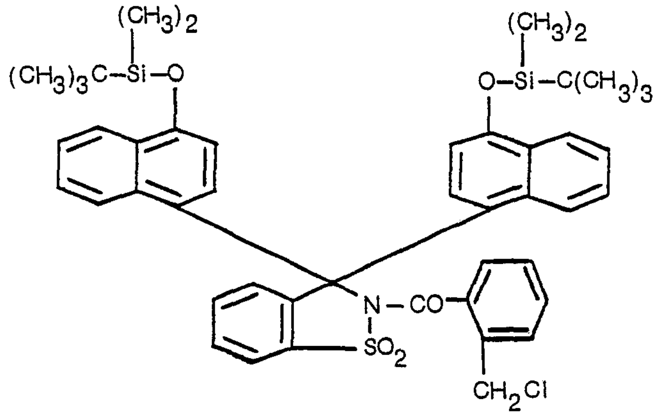

The 3-(4′-OP-1′-phenyl/4′-OP-1′-naphthyl)-3-(phenyl/naphthyl)-2,3-dihydrobenz[d]isothiazole-1,1-dioxides of this invention, i.e. those compounds of Formula (I) where R is hydrogen may be synthesized by reacting a 3-(4′-OP-1′-phenyl/4′-OP-1′-naphthyl)benz[d]-isothiazole-1,1-dioxide and a phenyllithium or a naphthyllithium reagent. Specifically, the 3-(4′-OP-1′-phenyl/4′-OP-1′-naphthyl)-benz[d]isothiazole-1,1-dioxides are prepared by blocking the functional hydroxy group and any substituent group (s), as may be appropriate, of the selected 4-halophenol or 4-halo-1-naphthol and converting the blocked phenol or 1-naphthol to the corresponding Grignard or lithium reagent which is then reacted with a saccharin reagent. The 4-halo substituent may be chloro, bromo or iodo when the lithium reagent is prepared by reacting the blocked phenol or blocked 1-naphthol with lithium metal and is either bromo or iodo when the lithium reagent is made via a lithium ion exchange resin using, for example, n-butyllithium. In preparing the Grignard reagent by reacting the blocked phenol or 1-naphthol with magnesium metal, the 4-halo substituent may be chloro, bromo or iodo. The Grignard or lithium reagent thus prepared is then reacted with saccharin, the N-lithium salt of saccharin or saccharin pseudo-chloride to yield the corresponding 3-(4′-OP-1′-phenyl/4′-OP-1′-naphthyl)benz[d]-isothiazole-1,1-dioxide. Generally, the Grignard reagent is reacted with the pseudo-chloride, and the lithium reagent is reacted with the N-lithium salt. The 3-(4′-OP-1′-phenyl/4′-OP-1′-naphthyl))-naphtho[1,8-de]-1,2-thiazine-1,1-dioxides may be prepared in a similar manner by reacting the Grignard or lithium reagent with 2,3-dihydro-3-oxo-naphtho[1,8-de]-1,2-thiazine-1,1-dioxide, its pseudochloride or the N-lithium derivative thereof.

To prepare the 3-(4′-OP-1′-phenyl/4′-OP-1′-naphthyl)-3-(phenyl/naphthyl)-2,3-dihydrobenz[d]-isothiazole-1,1-dioxides, the 3-(4′-OP-1′-phenyl/4′-OP-1′naphthyl)benz[d]-isothiazole-1,1-dioxide is reacted with at least one molar equivalent of a phenyllithium or a naphthyllithium reagent in an inert organic solvent, such, as benzene, diethyl ether, dioxane, hexane, toluene, petroleum ether or tetrahydrofuran. The reaction temperature may vary over a relatively wide range from about —80° to 50°C as may be readily determined for the particular reactants. For achieving maximum yields, the reaction generally is conducted at a temperature below about 0°C and preferably between about —65°C and —25°C.

The phenyl- or naphthyllithium reagent ultimately forming the B moiety of the subject compounds and the ultimate products may be substituted or unsubstituted and may be prepared from the corresponding halo-substituted compound. For example, N,N-dimethylaniline may be halogenated to give the 4-halo compound which, in turn, is reacted with lithium metal or n-butyllithium to yield the 4-lithium compound. Halogenation may be carried out in a conventional manner using either chlorine or bromine, with or without a catalyst, or using N-bromosuccinimide or iodinemonochloride. When lithium metal is employed in the preparation of the 4-lithium compound, the halo substituent may be chloro, bromo or iodo and is either bromo or iodo when a lithium exchange reaction is employed. If

substituents, such as, hydroxy, are present, they should be blocked with the appropriate protecting group to render them compatible with organometallic reagents prior to conversion to the 4-lithium compound. The protecting groups, of course, should be removable under mildly acidic conditions so that the substituents on the aryl moiety forming the B moiety can be regenerated simultaneously with the regeneration of the functional —OH group of the phenolic or naphtholic moiety, i.e. the A moiety.

The groups selected for protecting the functional phenolic or naphtholic hydroxy group and other hydroxy groups that may be present in the phenol or 1-naphthol should be stable to and compatible with organolithium and Grignard reagents and should protect the hydroxy group(s) against reaction under the conditions encountered in the synthesis of the aforesaid compounds and in the subsequent N-acylation step. In addition, the protecting group selected should be capable of being easily removed under weakly acid conditions to regenerate the hydroxy group(s) without the removal of or adversely affecting the N-carbonyl moiety or other substituents that may be present. Such protecting groups are selected from methoxymethyl, 2′-tetrahydropyranyl or dimethyl-t-butylsilyl. The blocked phenols and 1-naphthols employing these protecting groups may be prepared by methoxymethylation as described, for example, by Kaoru Fuji et al, Synthesis, 4, pp. 276—277 (1975), by tetrahydropyranylation as described, for example, by William E. Parham et al, J.Amer.Chem.Soc., 70, pp. 4187—4189 (1948) or by silylating with dimethyl-t-butylsilyl chloride in the presence of imidazole as described by E. J. Corey et al., J.Amer.Chem.Soc., 94, pp. 6190—6191 (1972).

As noted above, hydroxy groups in addition to the functional —OH of the phenol and 1-naphthol may be blocked simultaneously with the functional hydroxy group, for example, by tetrahydropyranylation or methoxymethylation. Groups other than hydroxy that sould be protected may be blocked prior to or subsequent to the blocking of the functional —OH. For example, carboxy group(s) may be protected by reacting a carboxy-substituted 4-halophenol (or 4-halo-1-naphthol) with 2-amino-2-methyl-1-propanol followed by blocking of the functional —OH. Sulphonamido (—NH—$SO_2$—R°) and sulfamoyl (—$SO_2$—NH—R°) substituents may be protected by a t-butyl group (R° is alkyl, aryl, alkaryl or aralkyl).

The 3-(4′-OP-1′-phenyl/4′-OP-1′-naphthyl)-3-phenyl/naphthyl)-2,3-dihydrobenz[d]isothiazole-1,1-dioxides prepared as described above are reacted with a carboxylic acid halide,

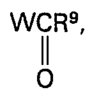

wherein W is chloro or bromo and wherein $R^9$ is selected from methyl, methyl substituted with at least one halogen atom selected from chloro, bromo and fluoro, alkoxy having 1 to 4 carbon atoms, phenyl, phenyl substituted in the para position with alkyl having 1 to 4 carbon atoms or —N,N-(dialkyl)-amino, phenyl substituted with at least one electron-withdrawing group, phenoxy, phenoxy substituted with at least one electron-withdrawing group, —$O(CH_2)_2Y$ wherein Y is an electron-withdrawing group and phenyl substituted in the ortho position with —$CH_2R^{10}$, wherein $R^{10}$ is chloro or bromo, preferably in pyridine solution to give the corresponding

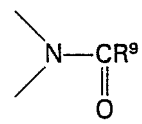

compound. About 1 to 2 moles of acid halides are used for each mole of the isothiazole-1,1-dioxide. Since the reaction is exothermic, external heating may not be necessary, but the reaction mixture may be heated to facilitate completion of the reaction, if desired. Ordinarily, the reaction temperature ranges between about 0° and 100°C, and preferably, the reaction is conducted in an inert atmosphere, for example, under nitrogen.

Optionally, the acylation step may be carried out by first reacting the isothiazole-1,1-dioxide with a molar equivalent and usually a slight excess of about 0,1 mole of an alkali metal hydride in an inert organic solvent at a temperature between about 0° and 100°C, preferably in an inert atmosphere, and then reacting the N-alkali metal salt thus formed with the said carboxylic acid halide,

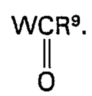

Usually, the acid halide is added to the reaction mixture containing the N-alkali metal salt. However, the N-alkali metal salt may be isolated prior to reaction with the acid halide, if desired. Solvents suitable for use in the alternate method of forming the

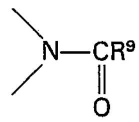

compound include dioxane, tetrahydrofuran, ethylether and benzene. The alkali metal, like the pyridine, affords substitution of the ring nitrogen of the ring-closing moiety in the acylation reaction.

Carboxylic acid halides are well known and may be prepared in a conventional manner, for example, by reacting the selected carboxylic acid, $R^9COOH$, with phosphorus trichloride, phosphorus pentachloride or thionyl chloride to give the corresponding $R^9COCl$, or by reacting the selected $R^9OH$ with phosgene to give the corresponding $ClCOOR^9$.

Subsequent to the acylation step, the protecting group P is removed from the functional —OH by treating with acid having a pH between about 0.1 and 5.0 at a temperature between about 20° and 100°C. The acid may be an inorganic acid, such as, hydrochloric acid or sulfuric acid in a protic solvent, e.g., water, alkanol, such as, methanol or ethanol, or aqueous alkanol, or the acid may be an organic acid, such as acetic acid or trifluoroacetic acid alone or in a protic solvent, such as those mentioned above. As indicated previously, any other protecting groups that may be present are removed simultaneously with the protecting group on the functional —OH.

The following examples are given to further illustrate the present invention and are not intended to limit the scope thereof. The examples also show how the intermediates comprising protected —OH groups which are the compounds according to the present invention, can be converted into compounds comprising functional —OH groups which from the subject matter of other applications.

EXAMPLE 1

Preparation of the compound having the formula:

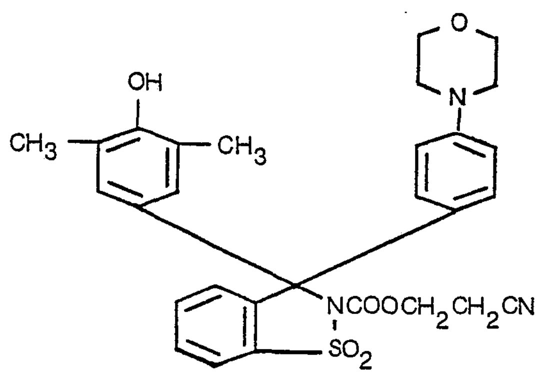

(a) N-(p-bromophenyl)morpholine (0.4 g.) was added to 20 ml. of tetrahydrofuran (THF) and the solution cooled to −65°C. To the solution was added 0.69 ml. of 2.4 M. butyllithium in hexane with stirring and stirring was continued for 1 hour. (After 15 minutes the solution became cloudy and a white precipitate formed.) To this solution was added 0.5 g. of 3-(3′, 5′-dimethyl-4′-methoxy-methoxy-1′-phenyl)benz[d]-isothiazole-1,1-dioxide in 2 ml. THF at −65°C under nitrogen. The resulting reaction mixture was a clear orange-yellow solution. The reaction mixture was stirred for 1 hour, poured into 100 ml. of water, made acidic with conc. hydrochloric acid (pH 6), and extracted with ether. The ether was dried over $Na_2SO_4$ and evaporated leaving an oil. The oil was taken up in ligroin (boiling range 30—60°C) and refluxed for 1 hour. The white solid that formed was collected to give 0.7g. of 3-(3′, 5′-dimethyl-4′-methoxymethoxy-1′-phenyl)-3-(4″-N-morpholinyl-1″-phenyl)-2,3-dihydrobenz[d]-isothiazole-1,1-dioxide having the formula:

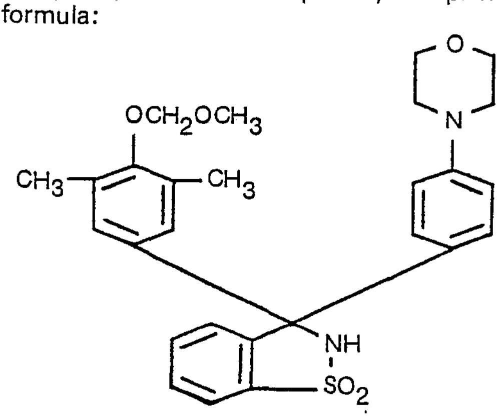

(b) The compound prepared in step (a) (0.7 g.) was placed in 20 ml. of pyridine under nitrogen and 0.15 ml. of β-cyanoethylchloroformate ($ClCO_2CH_2CH_2CN$) was added to the pyridine solution. The resulting reaction solution was stirred 1 hour, warmed gently and then poured into 100 ml. of water and extracted with chloroform. The chloroform was dried over $Na_2SO_4$, evaporated and the solid that formed was extracted with ligroin (boiling range 30—60°C). The solid obtained was the N-acylated compound, 3 - (3', 5' - dimethyl - 4' - methoxymethoxy - 1' - phenyl) - 3 - (4'' - N - morpholinyl - 1'' - phenyl) - 2 - (β - cyanocarbethoxy) - 2,3 - dihydrobenz[d]isothiazole - 1,1 - dioxide having the formula:

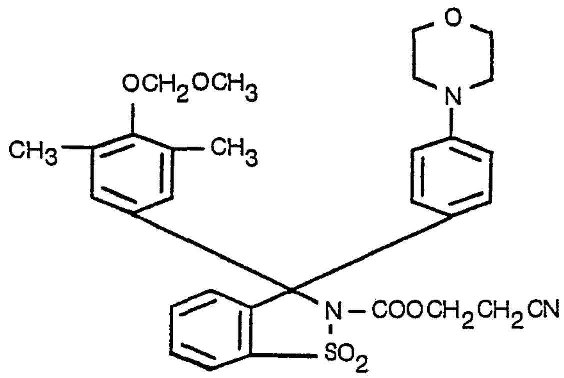

(c) The compound obtained in step (b) was then dissolved in methanol, made acidic with conc. hydrochloric acid and refluxed 1 hour. TLC from ether on silica gel showed 4 spots. The methanol solution was evaporated to leave 0.6 g. of solid. 200 mg. of solid in ether was placed on silica gel 1000 plates and the dark band was removed after drying plates. Acetone was used to remove the N-acylated product from the silica gel. The acetone was removed, ether added and the solution refluxed. The white solid that formed was recovered by filtration to give the title compound.

The 3-(3'-,5'-dimethyl-4'-methoxymethoxy-1'-phenyl)-benz[d]isothiazole-1,1-dioxide having the formula:

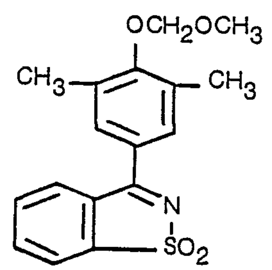

used in step (a) above was prepared as follows:

(i) Into a 2 liter three neck flask, fitted with a mechanical stirrer, nitrogen inlet and a dropping funnel, was placed 700 ml. of dry chloroform. The flask was immersed in an ice-water bath. Powdered phosphorus pentoxide (300.0 g.) was added to the vigorously stirred, cold chloroform. To this mixture was added over a 1 hour period a solution of 4-bromo-2,6-dimethylphenol (201.0 g.) in 400 ml. of dry dimethoxymethane. During this time the phosphorus pentoxide powder fused into an amorphous mass and stirring became difficult. TLC analysis (9:1 petroleum ether-ethyl acetate on silica gel) indicated that much unreacted starting phenol was still present. The temperature of the reaction mixture was allowed to rise to about 25°C. Additional 50 g. increments of phosphorus pentoxide were added to the stirred reaction mixture every 30—45 minutes until TLC analysis indicated the absence of starting phenol. The organic layer was decanted, washed with two 250 ml. portions of aqueous 10% sodium hydroxide and dried over calcium sulfate. The solvent was removed under reduced pressure leaving a pale yellow oil which was distilled from 25 g. of anhydrous potassium carbonate to give 220.0 g. of 4-bromo-2,6-dimethyl-methylenemethoxyphenyl ether as a colorless oil [boiling point 112°C and 0.67 millibars (0.5 mm Hg)].

(ii) 4-Bromo-2,6-dimethyl-methylenemethoxyphenyl ether (85.04 g; 0.347 mole) was dissolved in approximately 800 ml of tetrahydrofuran. The solution was cooled to −75°C under a nitrogen blanket, and 2.4M n-butyllithium in hexane (144.8 ml; 0.346 mole) was added dropwise. Addition was completed within a 2 hour period giving a white slurry.

(iii) Saccharin (61.2 g; 0.334 mole) was dissolved in 600 ml. of dry tetrahydrofuran, and the solution was cooled to approximately −75°C. 2.4M n-butyllithium in hexane (130.4 ml; 0.311 mole) was slowly added dropwise to the cooled solution under a nitrogen blanket. The temperature was not allowed to rise above −70°C. Addition was completed in about 90 minutes, giving a clear, very pale yellow solution.

(iv) The yellow solution obtained in step (iii) was slowly added (over a 3 hour period) to the white slurry obtained in step (ii) while keeping the temperature at −70°C. During this time the solids disappear giving a clear, caramel colored reaction mixture that first tends to darken whith time and then gradually lightens. The reaction mixture was allowed to come to room temperature overnight and then was treated with 36.0 g. of ammonium chloride in 250 ml. of water, while cooling in an ice-water bath. The organic portion was decanted and dried over anhydrous calcium sulfate. The solvent was removed under reduced pressure to give a pink colored oil that became solid on standing in open air.

The solid was recrystallized twice from 1-propanol, washed with a 60:40% mixture of petroleum ether-tetrahydrofuran and dried under vacuum to give 68.0 g. of the title compound as a white, crystalline solid.

3-(3′,5′-dimethyl-4′-methoxyethoxy-1′-phenyl)-benz[d]isothiazole-1,1-dioxide also was prepared as follows:

Dry tetrahydrofuran (10—15 ml.) was added to magnesium turnings (0.20 g.) under nitrogen. A solution of 4-bromo-2,6-dimethyl-methylenemethoxyphenyl ether (2.0 g.) in tetrahydrofuran (30 ml.) was added gradually to the magnesium turnings with stirring and heating. After about twenty minutes of external heating to reflux, a self-sustaining reaction was observed. The remaining solution of phenyl ether was then added at a rate to maintain a comfortable reaction. Refluxing with external heating was continued after addition was complete and after one hour, the solution was cooled to room temperature and held under nitrogen. A solution of saccharin pseudochloride (1.89 g.) in tetrahydrofuran (40 ml.) was cooled to −78°C and the previously prepared solution of magnesium bromide reagent was added dropwise to the pseudo-chloride solution under nitrogen. The resulting reaction mixture was stirred cold for about 2 hours and then stirred at room temperature overnight. The reaction mixture was then cooled in an ice water bath and treated with saturated aqueous ammonium chloride solution. The aqueous solution was extracted with chloroform several times and the combined chloroform extracts washed with water and dried over anhydrous sodium sulfate followed by drying over anhydrous calcium sulfate. Upon removing the chloroform, a colorless oil was obtained which was extracted several times with small portions of light petroleum ether to leave behind a pale yellow tacky tar. The yellow tar was treated with ether leaving behind an off-white solid. The off-white solid was dissolved in a small amount of chloroform, treated with carbon black and filtered through a silica filter aid. Upon removing the solvent, the title compound was obtained as an off-white solid which was dried under vacuum in the presence of $P_2O_5$. Yield 0.520 g.

3-chlorobenz[d]isothiazole-1,1-dioxide (saccharin pseudochloride) was prepared as follows:

35 g. of saccharin and 43.7 g of $PCl_5$ were heated at 170°C for 1 1/2 hours during which time complete solution occurred and $POCl_3$ began to reflux. The $POCl_3$ was removed at reduced pressure to leave a crystalline residue. Di-ethyl ether was added to the crystalline residue and stirred well. The title compound was recovered as white crystals, 12.5 g. (melting range 146°—147°C).

The N-(p-bromophenyl)morpholine having the formula

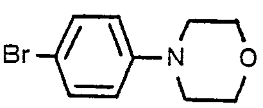

used in step (a) above was prepared as follows:

25 g. of N-phenylmorpholine was dissolved in 200 ml. of carbon tetrachloride and stirred well. To this was added all at once 27.2 g. of N-bromosuccinimide. There was an exotherm to 45°C. The reaction solution was stirred until the temperature began to decrease and then was heated to reflux for 3 hours. TLC on silica gel with 3/2 petroleum ether/ether indicated that the reaction was complete. The reaction solution was then cooled, the succinimide removed by filtration and the solution evaporated to yield a yellow solid. The solid was dissolved in 250 ml of ethanol and cooled to give 22 g. of the title compound as white crystals.

The β-cyanoethylchloroformate having the formula ($CNCH_2CH_2COOCl$) used in step (b) above was prepared as follows:

To 100 ml of dry benzene, cooled in an ice bath, was added phosgene gas until 34.0 g. was collected. Hydroxyacrylonitrile (20.2 g.) was added to the cooled phosgene solution. (The temperature rose slightly to approximately 8°C). The resulting heterogeneous mixture was cooled to 3°C with stirring, and pyridine (22.6 g.) in 25 mls. of benzene was added dropwise. Heat was evolved, and the temperature was not allowed to rise above 10°C. Very vigorous stirring was maintained until solid began to form. After about 1 hour, the reaction mixture was stirred at approximately 5°C for 15 minutes, then allowed to come to 15°C—20°C and stirred for another 15 minutes. The reaction mixture was then cooled to 5°C and 26 ml. of ice water was added in increments. The solids dissolved with liberation of heat and evolution of gas. The temperature was not allowed to rise above 15°C over the next 15—20 minutes. The reaction mixture was then stirred at room temperature for 2 hours, the benzene layer decanted and dried over anhydrous $Na_2SO_4$, followed by drying over anhydrous $CaSO_4$. The solvent was removed under reduced pressure to yield an almost colorless oil which was redistilled

under vacuum at a boiling range of 68.5°—70.5°C (pot temperature 98—103°C) to yield 18.2 g. of the title compound as a colorless oil.

Examples 2 and 3

The compounds having the formulas

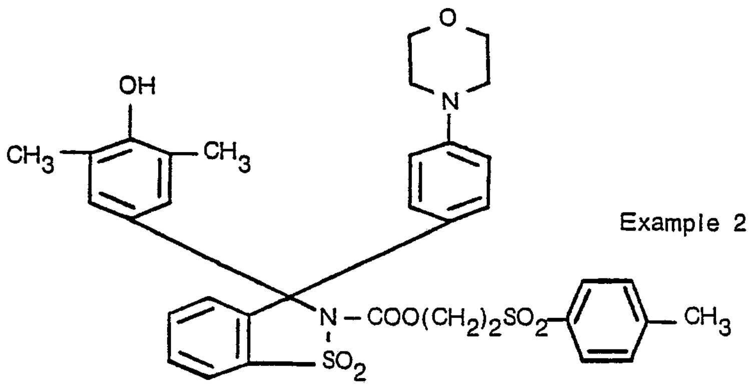

Example 2

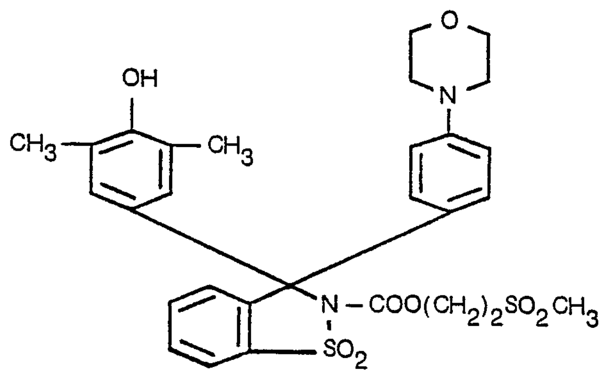

Example 3

were prepared in the same manner described in Example 1 except that the 3-(3′,5′-dimethyl-4′-methoxymethoxy-1′-phenyl)-3-(4″-N-morpholinyl-1″-phenyl)2,3-dihydrobenz[d]isothiazole-1,1-dioxide was reacted in step (b) with the appropriate acylating agents, namely,

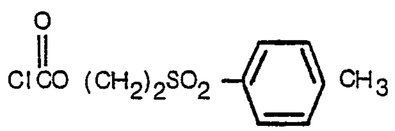 and 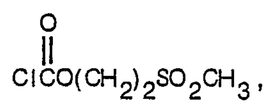,

to yield the corresponding N-acylated compounds which were treated as in step (c) above to remove the protecting groups.

EXAMPLE 4

Preparation of the compound having the formula

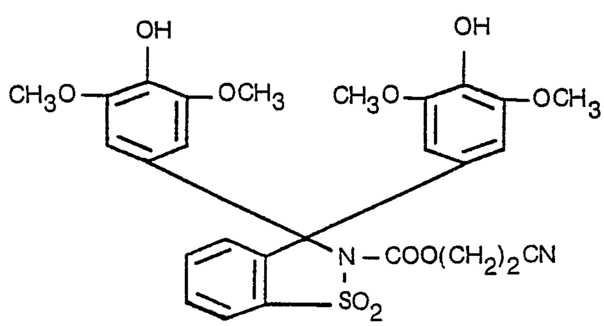

(a) 55 g. of 4-bromo-2,6-dimethoxy-methylenemethoxyphenyl ether was dissolved in 300 ml. of anhydrous tetrahydrofuran under a stream of nitrogen. The solution was cooled to —65°C during which time some of the phenyl ether precipitated. To this was added 79 ml of butyl-lithium (2.4M in hexane) at a range to keep the temperature below —50°C. The resulting solution was cooled to —65°C and was stirred for 30 minutes. To this solution was added 19 g. of sacharin pseudo-chloride in two portions so as to keep the temperature below —40°C. The reaction solution was cooled to —65°C and was stirred for 40 minutes. TLC showed one main spot on silica gel with 10 ml. ether/2 drops methanol. The reaction solution was poured into 2000 ml. of water and made acidic to pH 6. The mixture changed color from orange to yellow at this pH. The mixture was extracted two times with ether (2 liters) and the ether washed with water. The ether was dried over sodium sulfate and evaporated to leave a light yellow solid. the solid was recrystallized from 450 ml. of n-propanol to give 42 g. of 3,3-di(3′,5′-dimethoxy-4′-methoxymethoxy-1′-phenyl)-2,3-dihydrobenz[d]isothiazole-1,1-dioxide as off-white crystals (melting range 151.5°—152.5°C).

(b) The compound prepared in step (a) (27.0 g) was dissolved in 125 ml. of dioxane at room temperature under nitrogen. To this solution was added 2.50 g. of NaH (57% oil dispersion) and the resulting dispersion stirred for 45 minutes. (Evolution of hydrogen was observed.) Then 8.0 ml. of $ClCOOOCH_2CH_2CN$ was added and an exotherm resulted. The resulting reaction mixture was stirred for 3 hours. The initial yellow color disappeared and a white dispersion formed. The dispersion was poured into 2 liters of water, made neutral with dilute HCl and extracted with chloroform. The chloroform was washed with water, separated, dried over sodium sulfate, and evaporated to yield a white solid. The solid was stirred with ethyl ether, isolated by filtration, dried in vacuo, and crystallized from methanol. The needles that formed were recovered by filtration to give the $>N—COOCH_2CH_2CN$ derivative of 3,3-di(3′,5′-dimethoxy-4′-methoxymethoxy-1′-phenyl)-2,3-dihydrobenz[d]isothiazole-1,1-dioxide.

(c) 3.7 g. of 3,3-di(3′,5′-dimethoxy-4′-methoxy-methoxy-1′-phenyl)-2-β-cyanocarbethoxy-2,3-di-hydrobenz[d]-isothiazole-1,1-dioxide prepared as in step (b) above was placed in 200 ml. of methanol and 2 drops of HCl were added. The resulting solution was refluxed and the reaction was followed by TLC on silica gel with ether. When TLC indicated that no more blocked compound remained, the reaction was discontinued and the methanol removed in vacuo to leave an egg-white solid (3.2 g). This was dissolved in 80 ml. of 1,2-dichloroethane with heating and 80 ml. of petroleum ether was added with swirling. The solution was cooled and the light beige crystals that formed were collected and dissolved in 175 ml. of ethanol at reflux. The ethanol solution was allowed to cool in the refrigerator overnight and 2.7 g. of the title compound was collected as white needles (melting range 191°—193°C).

The methoxymethylation of 4-bromo-2,6-dimethoxy-phenol used in step (a) above was carried out as follows:

To a 3 liter flask was added 300 g. of $P_2O_5$ under nitrogen and 800 ml. of chloroform (previously dried over $P_2O_5$). The mixture was cooled to —15°C with a dry-ice acetone bath and then 50 g. of 4-bromo-2,6-dimethoxyphenol in 800 ml of dimethoxymethane was added over a 25 minute period while maintaining the temperature at —15°C or below. To the resulting reaction mixture was added 1 ml. of conc. sulfuric acid and then the temperature was allowed to come to room temperature. During this time, a tacky mass of $P_2O_5$ developed. The reaction mixture was stirred for 3 hours. TLC indicated that the reaction was complete. The chloroform was then decanted into 400 ml. of 10% aqueous sodium hyroxide, stirred well and the chloroform layer separated, washed with water, dried over $Na_2SO_4$ and evaporated to leave light tan crystals. n-Propanol was added to the crystalline residue, stirred and filtered to give 32.7 g. of the title compound as white crystals (melting range 98°—100°C).

Examples 5 and 6

The compounds having the formulas

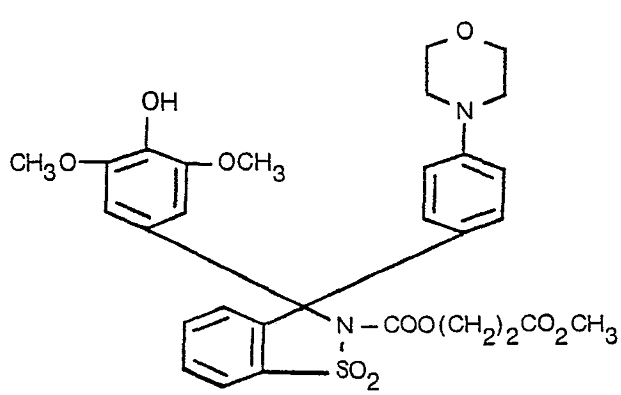

Example 5

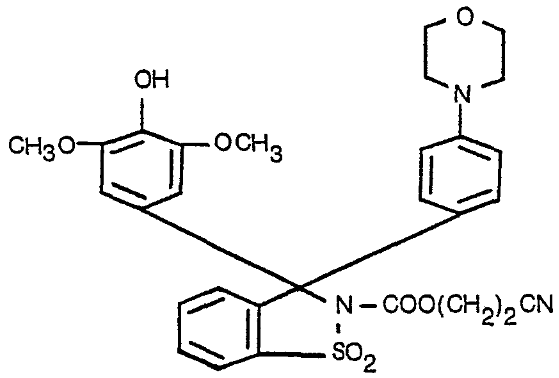

Example 6

were prepared according to the procedure described in Example 1 except that 3-(3′,5′-dimethoxy-4′-methoxymethoxy-1′-phenyl)-benz[d]isothiazole-1,1-dioxide was used in the reaction of step (a) and the appropriate acylating agents namely,

$$ClC(=O)O(CH_2)_2SO_2CH_3 \quad \text{and} \quad ClC(=O)O(CH_2)_2CN$$

were used in step (b).

The 3-(3′,5′-dimethoxy-4′-methoxymethoxy-1′-phenyl)-benz[d]isothiazole-1,1-dioxide used in step (a) above was prepared as follows:

(i) Using an 18 gauge needle-syringe, 20.0 mls. of n-butyllithium (2.4M in hexane) was added dropwise over 1 hour to a solution of 9.16 g. of saccharin (previously dried overnight at 80°C in vacuo) in 250 mls. of dry tetrahydrofuran under nitrogen at —75°C to —73°C with rapid stirring. The reaction solution comprising the N-lithium salt of saccharin in tetrahydrofuran was used directly in step (iii) without isolating the lithium salt.

(ii) In a dried 1 l. flask, 13.86 g. of 4-bromo-2,6-dimethoxy-methylenemethoxyphenyl ether was dissolved in 300 mls. of dry tetrahydrofuran under nitrogen, and 20.83 mls of n-butyllithium (2.4M in hexane) was added dropwise with stirring at —75°C. After addition was complete, the reaction solution was stirred at —75°C for about 30 minutes.

(iii) The solution of saccharin lithium salt prepared in step (i) was transferred to an addition funnel using a double-tip needle under nitrogen pressure and added to the solution of 4-Li-2,6-dimethoxy-methylenemethoxyphenyl ether prepared in step (ii) over about 15 minutes with stirring at a temperature of —75°C to —70°C. The reaction mixture was stirred for about 2 hours at —75°C and then warmed to 0°C during an hour.

(iv) A solution of 5.2 g. of ammonium chloride in 175 mls. of water was added dropwise to the reaction mixture of step (iii) and the reaction mixture was transferred to a 1 l. separatory funnel. After the two phases separated, the aqueous phase was removed and the pH was lowered from about 11 to about 6—7 by the dropwise addition of aqueous 5% hydrochloric acid solution. (A color change from yellow to colorless was observed). The aqueous phase was returned to the separatory funnel, partitioned and the aqueous phase again separated and then extracted with fresh ether (100 mls.). The ether and tetrahydrofuran/hexane extracts were combined, dried over magnesium sulfate overnight, filtered and the solvent removed to leave a yellow oil which crystallized. Ether (100 mls.) was added to the crystalline material and the crystalline material was ground under ether in a mortar, filtered, washed with more ether followed by petroleum ether and air dried. A second crop was collected from the filtrate to give a total yield of 13.0 g. of the title compound.

## Examples 7—11

The compounds having the formulas:

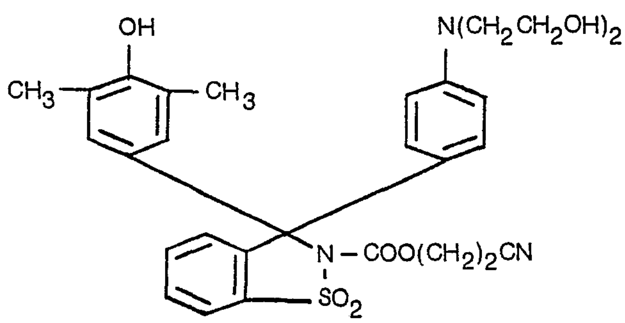

Example 7

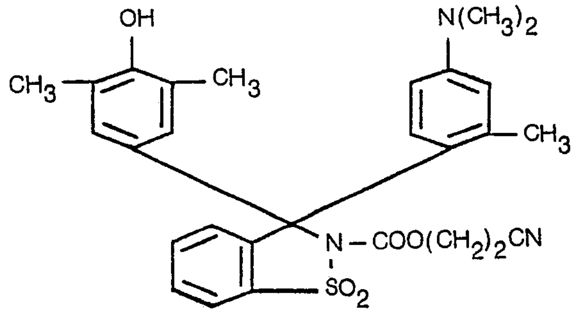

Example 8

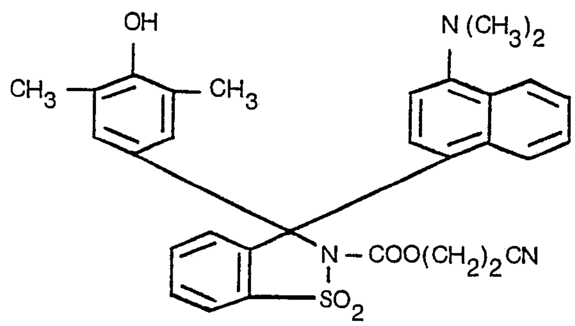

Example 9

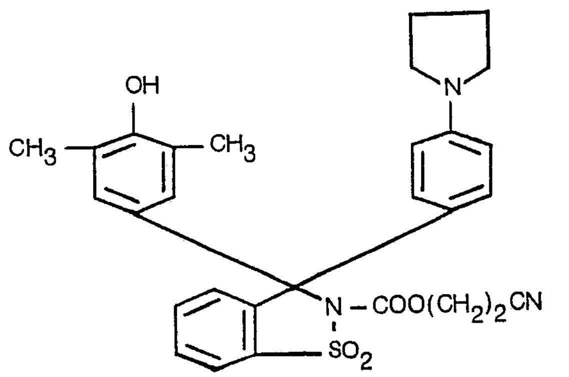

Example 10

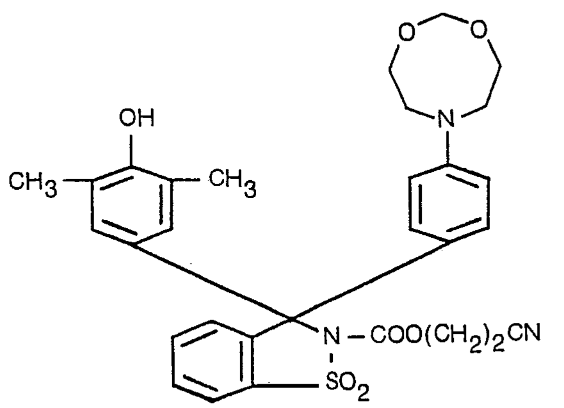

Example 11

were prepared according to the procedure given in Example 1 except that in step (a) the 3-(3′,5′-dimethyl-4′-methoxymethoxy-1′-phenyl)benz[d]isothiazole-1,1-dioxide was reacted with the appropriate lithium reagent, namely,

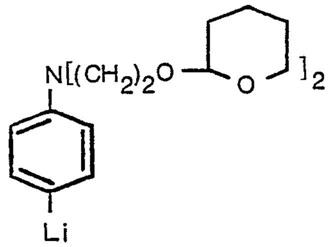 ; 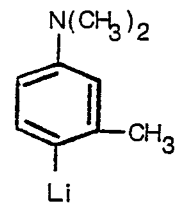 ; 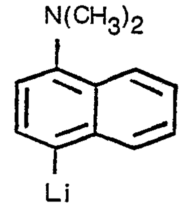 ;

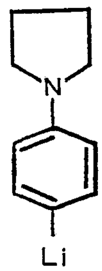 ; and 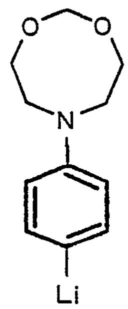 , respectively,

and the 2′-tetrahydropyranyl groups were removed from the protected N,N-di(β-hydroxyethyl)anilino moiety of Example 7 in step (c) simultaneously with the 4′-methoxymethyl protecting group.

N-(p-Li-phenyl)tetrahydro-2H,4H-1,3,6-dioxazocine may be prepared by reacting the corresponding p-halophenyl compound with n-butyllithium. The N-(p-halophenyl)tetrahydro-2H,4H-1,3,6-dioxazocines are prepared by reacting p-halo-N,N-di(β-hydroxyethyl)aniline with certain dihalomethanes in the presence of a solid alkali metal hydroxide or concentrated aqueous solution thereof and a quaternary ammonium salt.

Tetrahydropyranylation of p-Br-N,N-di(β-hydroxyethyl)aniline was carried out as follows:

p-Br-N,N-di(β-hydroxyethyl)aniline (20.0 g.) was dissolved in 475 ml. of dichloromethane containing 60 ml. of dihydropyran. To this solution was added 1 ml. of conc. HCl, and the reaction solution was stirred for about 5.5 hours. The solution was then washed with water containing enough sodium hydroxide to neutralize any acid present. The dichloromethane was separated, dried over anhydrous sodium sulfate and evaporated under reduced pressure with a steam bath (aspirator) leaving an oil. The oil was heated to 115°C at 0.13 millibars (0.1 mm Hg) to distill off impurities leaving 33.0 g. of the title compound.

The corresponding 4-Li compound was prepared by dissolving 4-bromo-N,N-di(β-2′-tetrahydropyranyloxyethyl)aniline (10.0 g.) in 100 ml. of tetrahydrofuran, cooling the solution to −65°C and adding 10 ml. of n-butyllithium (2.4 M in hexane) under nitrogen at a rate to maintain the temperature at −65°C.

Examples 12—14

The compounds having the formulas

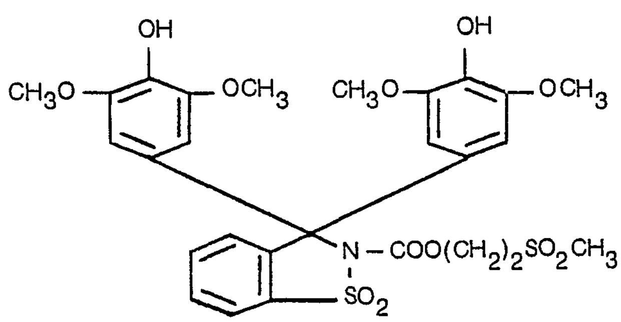

Example 12

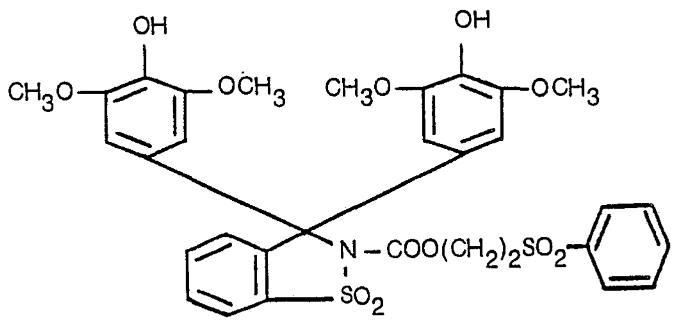

Example 13

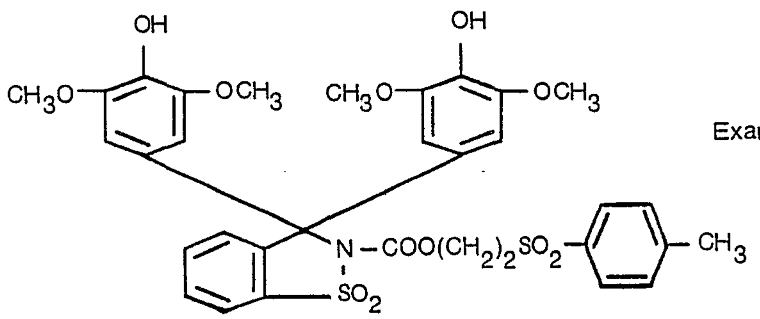

Example 14

were prepared in the same manner described in Example 4 except that the acylating agents employed were

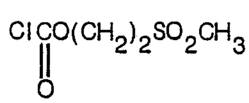, 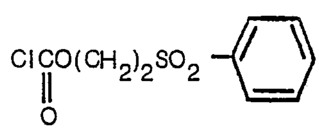 and

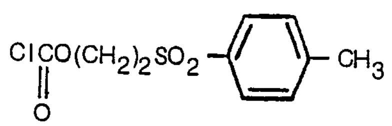, respectively

Example 15

Preparation of the compound having the formula:

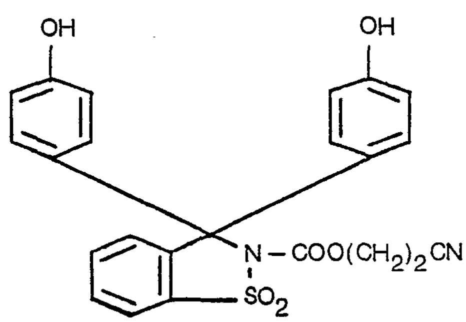

(a) 2'-tetrahydropyranyl 4-bromophenyl ether (9.0 g.) in 20 ml. of tetrahydrofuran was added dropwise to 0.85 g. of magnesium in 20 ml. of tetrahydrofuran under nitrogen. After addition was complete, the dispersion was refluxed for 2 hours and then saccharin pseudo-chloride (3.5 g.) was added portionwise. An exotherm was observed, and the green solution that formed turned yellow-brown. The reaction solution was stirred overnight and then slowly added to 500 ml. of water. The precipitate that formed was slowly filtered and the solid obtained was dissolved in ether, dried and evaporated to yield 8.5 g. of light yellow solid having the formula:

A

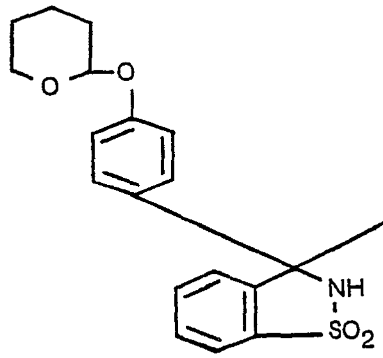

(b) Compound A as prepared in step (a) (10.0 g.) was placed in 100 ml. of dioxane and 0.88 g. of sodium hydride (as 57% oil dispersion) was added at room temperature under nitrogen (hydrogen evolution was observed). The resultant mixture comprising the N-sodium salt of the above compound was stirred 1 hour and then 2.4 ml. of $ClCOOCH_2CH_2CN$ was added. A white precipitate formed immediately. The reaction mixture was stirred overnight then poured into 200 ml. water. The aqueous mixture was extracted with three 250 ml. portions of ether, which were combined and dried over sodium sulfate. The ether was removed *in vacuo* and the resulting solid washed with petroleum ether. Upon drying, 10.5 g. of the crude N-acylated derivative was obtained. The N-acylated compound has the formula

B

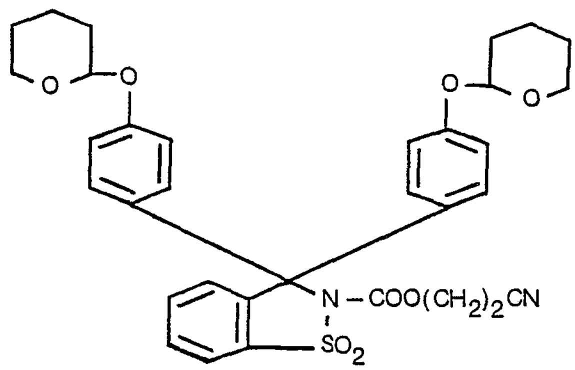

(c) Compound B (5.0 g.) as prepared in step (b) was dissolved in 50 ml. of warm methanol which contained two drops of conc. HCl. This solution was allowed to stand at room temperature for 30 min. at which time the analysis indicated both tetrahydropyranyl protecting groups had been removed. The methanol and HCl were removed *in vacuo* leaving 5.0 g. of a light yellow solid which was fractionated via column chromatography (silica gel; ether) to give a pure sample of the title compound.

Tetrahydropyranylation of p-bromo-phenol used in the preparation of compound A was carried out as follows:

To 10.5 ml. of dihydropyran containing 2 drops of conc. HCl was added 10.0 g. of p-bromophenol. (The reaction was exothermic; temperature rose to 35°C). After the addition was completed, the colorless solution obtained was heated to 50°C and allowed to cool with stirring for 1 hour. The solution was extracted with 20 ml. of ether and 10 ml. of 10% NaOH. The ether layer was dried over anhydrous $Na_2SO_4$ and evaporated under reduced pressure to leave an oil. 80 ml. of ethanol was added to the oil and the resulting ethanol solution was allowed to stand. The white crystals that formed were recovered by filtration and dried under vacuum to yield 7.3 g of the blocked phenol. The mother liquor was concentrated to one-half its original volume and cooled. More crystals formed which were isolated to yield an additional 2.1 g. of blocked phenol.

Example 16

Preparation of the compound having the formula:

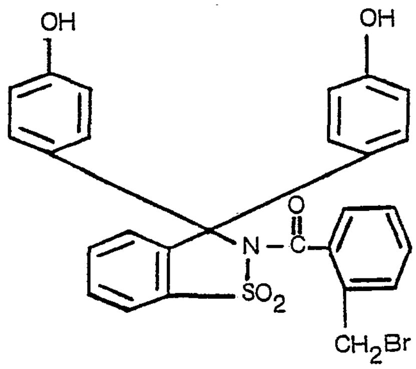

(a) 2′-tetrahydropyranyl 4-bromophenyl ether (9.0 g.) in 20 ml. of tetrahydrofuran was added dropwise to 0.85 g. of magnesium in 20 ml. of tetrahydrofuran under nitrogen. After addition was complete, the dispersion was refluxed for 2 hours and then saccharin pseudo-chloride (3.5 g.) was added portionwise. An exotherm was observed, and the green solution that formed turned yellow-brown. The reaction solution was stirred overnight and then slowly added to 500 ml. of water. The precipitate that formed was slowly filtered and the solid obtained was dissolved in ether, dried and evaporated to yield 8.5 g. of light yellow solid having the formula:

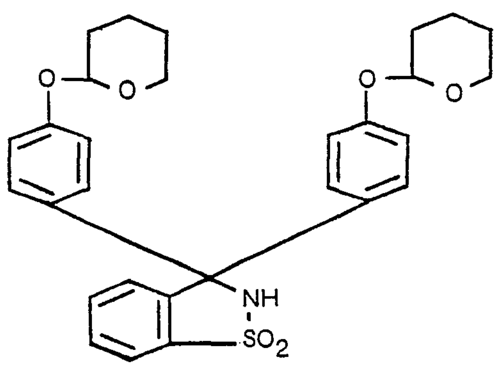

(b) 1.0 g. of the compound prepared in step (a) was placed in 15 ml. of dioxane and 0.08 g. of sodium hydride (as 57% oil dispersion) was added at room temperature under nitrogen (Hydrogen evaluation was observed). The resultant mixture comprising the N-sodium salt of the above compound was stirred for 1 hour and then 0.342 g. of

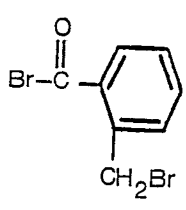

was added. A white precipitate formed immediately. The reaction mixture was stirred overnight, then poured into 100 ml. water. The white precipitate that formed was filtered, extracted with diethyl ether giving a yellow glass that was refluxed in ligroin (90°—100°C boiling range). The ligroin was decanted leaving a solid comprising the N-acylated derivative of the above compound wherein the acyl group is

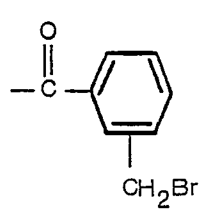

(c) To remove the 2′-tetrahydropyranyl protecting groups and obtain the product, 20 mls. of 10% HCl solution was added to the solid and the orange solid obtained was filtered and dried. A second crop

of precipitate was recovered from the water precipitation which was filtered and treated with 10% HCl to give an additional 0.5 g. of solid. The solids were combined and fractionated via preparative TLC (silica gel/ether) to give a pure sample of the title compound.

Example 17

Preparation of the compound having the formula

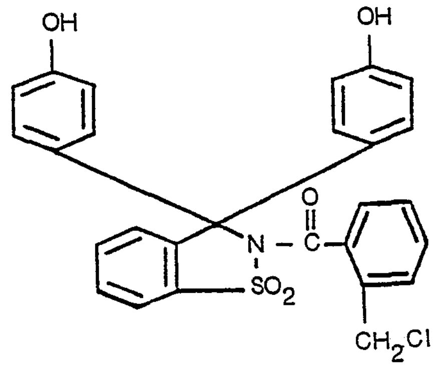

The title compound was prepared according to the procedure of Example 16 except that

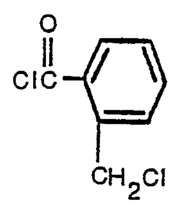

was employed as the acylating agent in step (b).

Example 18

Preparation of the compound having the formula:

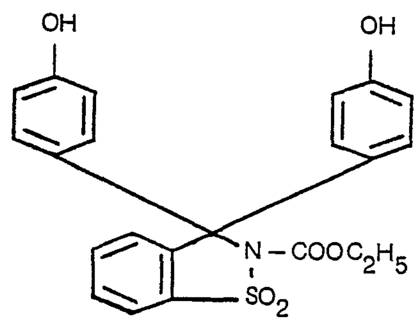

(a) 2′-tetrahydropyranyl 4-bromophenyl ether (9.0 g.) in 20 ml. of tetrahydrofuran was added dropwise to 0.85 g. of magnesium in 20 ml. of tetrahydrofuran under nitrogen. After addition was complete, the dispersion was refluxed for 2 hours and then saccharin pseudo-chloride (3.5 g.) was added portionwise. An exotherm was observed, and the green solution that formed turned yellow-brown. The reaction solution was stirred overnight and then slowly added to 500 ml. of water. The precipitate that formed was slowly filtered and the solid obtained was dissolved in ether, dried and evaporated to yield 8.5 g. of light yellow solid having the formula:

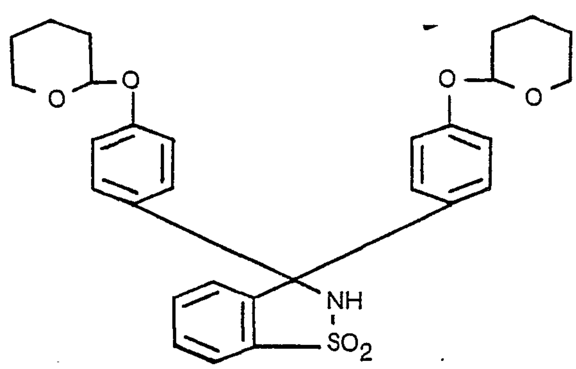

(A)

(b) The compound as prepared in step (a) (1.0 g.) was placed in 10 ml. of dioxane and 0.08 g. of sodium hydride (as 57% oil dispersion) was added at room temperature under nitrogen (Hydrogen evolution was slow). The solution went from light yellow to greenish then to yellow. The reaction was accelerated by heating to about 50°C and then allowing the solution to cool to room temperature, about 1 hour. To the remaining solution comprising the N-sodium salt of Compound A was added 0.18 ml. of ethylchloroformate and the reaction mixture was stirred over the weekend.

(c) To remove the protecting groups, the solution was poured into 20 ml. of conc. HCl. After stirring for 15 minutes, the solution went from pink-red to brown. The precipitate that formed was filtered and dried under vacuum. Crystallization from 1:1 methanol-water gave 0.5 g. of the title compound as a white solid.

Examples 19 and 20

Preparation of the compounds having the formula:

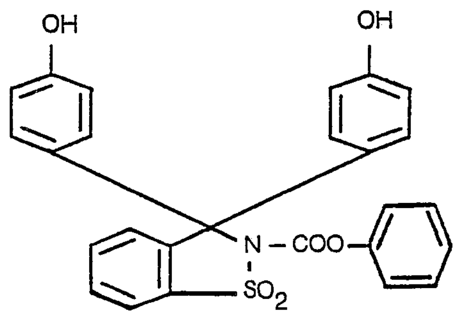

Example 19

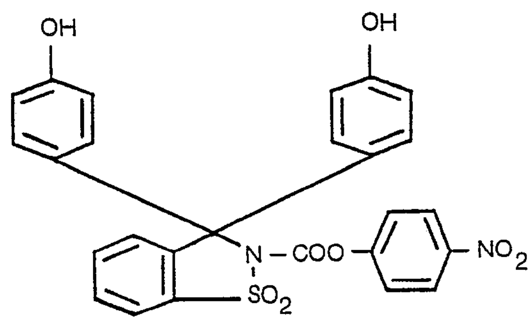

Example 20

The title compounds were prepared according to the procedure described in Example 18 above except that the acylating agents employed in step (b) were

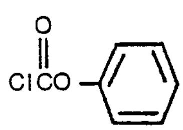

and

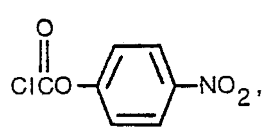

, respectively.

Example 21

Preparation of the compound having the formula:

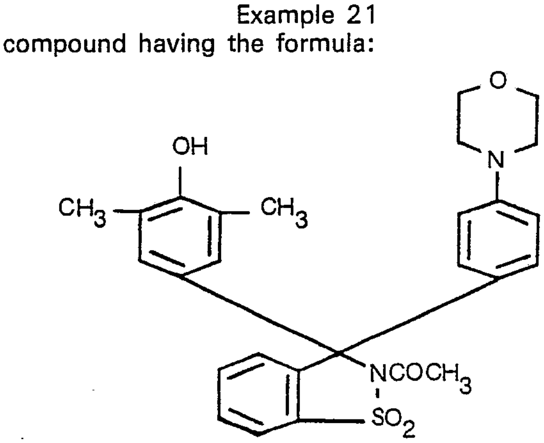

(a) N-(-p-bromophenyl)morpholine (0.4 g.) was added to 20 ml. of tetrahydrofuran (THF) and the solution cooled to –65°C. To the solution was added 0.69 ml. of 2.4 M butyllithium in hexane with stirring and stirring was continued for 1 hour. (After 15 minutes the solution became cloudy and a white precipitate formed). To this solution was added 0.5 g. of 3-(3′,5′-dimethyl-4′-methoxymethoxy-1′-phenyl) benz[d]-isothiazole-1,1-dioxide in 2 ml. THF at >65°C under nitrogen. The resulting reaction mixture was a clear orange-yellow solution. The reaction mixture was stirred for 1 hour, poured into 100 ml of water, made acidic with conc. hydrochloric acid (pH 6), and extracted with ether. The ether was dried over $Na_2SO_4$ and evaporated leaving an oil. The oil was taken up in ligroin (boiling range 30—60°C) and refluxed for 1 hour. The white solid that formed was collected to give 0.7 g. of 3-(3′,5′-dimethyl - 4′ - methoxymethoxy - 1′ - phenyl) - 3 - (4″ - N - morpholinyl - 1″ - phenyl) - 2,3 - dihydrobenz[d] - isothiazole - 1,1 - dioxide having the formula:

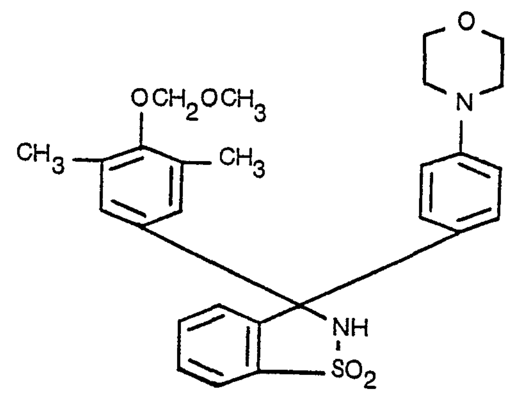

(b) The compound prepared in step (a) (0.7 g.) was dissolved in 15 ml. of pyridine under nitrogen and 0.11 ml. of

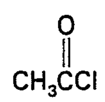

was added to the pyridine solution. A white precipitate formed. The resulting reaction solution was stirred and warmed gently (total stirring time 1 hour) and then poured into 100 ml. of water and extracted with chloroform. The chloroform was dried over anhydrous $Na_2SO_4$ and evaporated leaving a solid. The solid obtained was the N-acylated compound, 3-(3′,5′-dimethyl-4′-methoxymethoxy-1′-phenyl)-3-(4″-N-morpholinyl-1″-phenyl)-2-(acetyl)-2,3-dihydrobenz[d]isothiazole-1,1-dioxide having the formula:

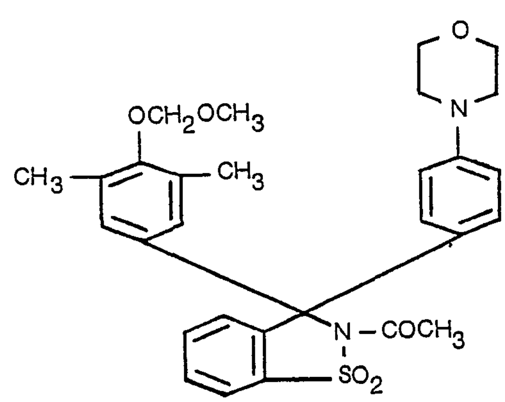

(c) The compound obtained in step (b) was then dissolved in 25 ml. of methanol, made acidic with conc. hydrochloric acid and refluxed 2 hours. The solution was evaporated leaving a tan solid. TLC of a sample on silica gel 1000 with ether gave a dark band on drying. The dark band was removed, washed with acetone and the acetone removed after filtering to yield a gum. Ether was added to the gum and the resulting solution refluxed to give a white solid that was isolated to give the title compound.

Examples 22—24

The compounds having the formulae

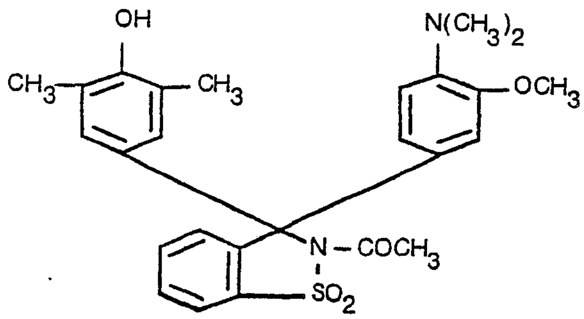

Example 22

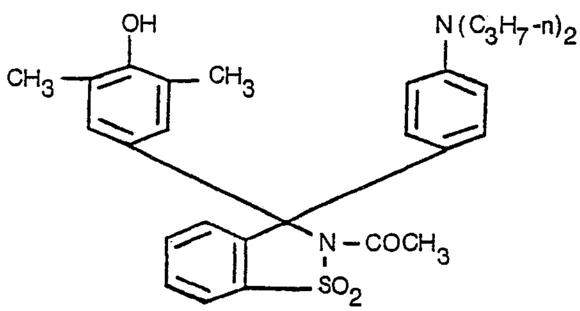

Example 23

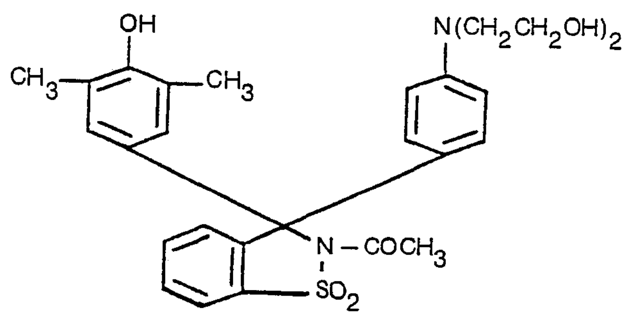

Example 24

were prepared according to the procedure given in Example 21 except that in step (a) the 3-(3′,5′-dimethyl-4′-methoxymethoxy-1′-phenyl)benz[d]isothiazole-1,1-dioxide was reacted with the appropriate lithium reagent namely,

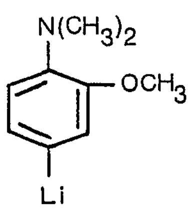

,

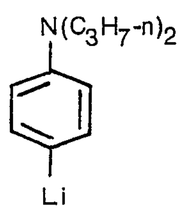

and

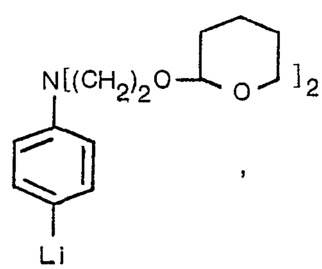

,

respectively, and the 2′-tetrahydropyranyl groups were removed from the -N,N-di(β-hydroxyethyl)anilino moiety simultaneously with the methoxymethyl-protecting group in step (c).

Examples 25 and 26

The compounds having the formulae

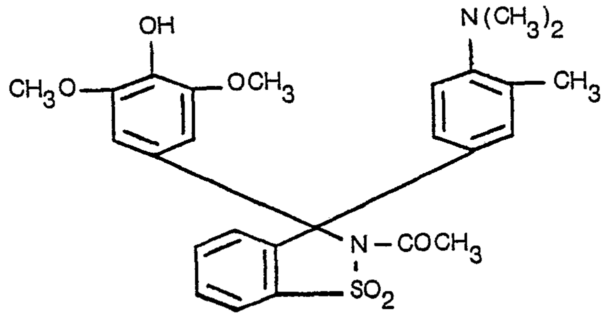

Example 25

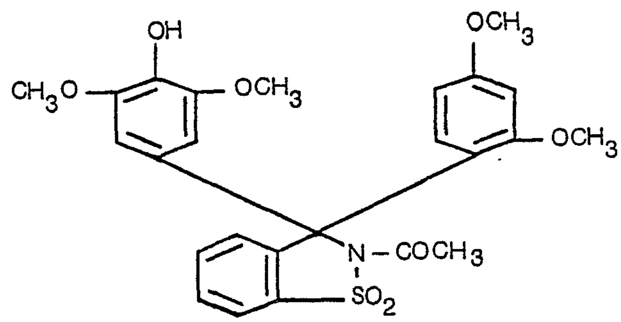

Example 26

were prepared according to the procedure given in Example 21 except that 3-(3′,5′-dimethoxy-4′-methoxymethoxy-1′-phenyl)-benz[d]isothiazole-1,1-dioxide was reacted with the appropriate lithium reagent, viz.

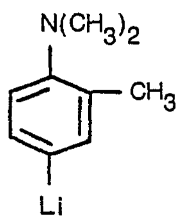

and

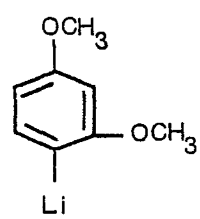

, respectively

Examples 27 and 28

Preparation of the compounds having the formulae:

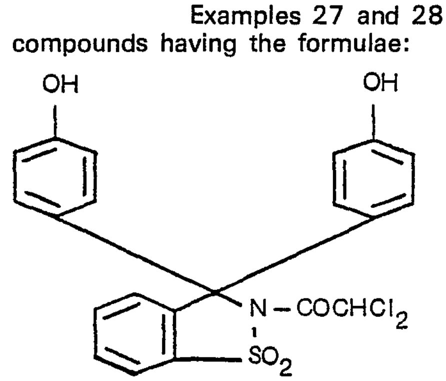

Example 27

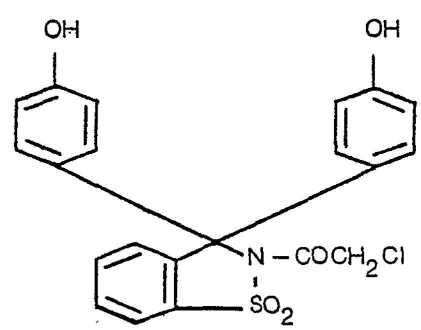

Example 28

(a) 2′-tetrahydropyranyl 4-bromophenyl ether (9.0 g.) in 20 ml. of tetrahydrofuran was added dropwise to 0.85 g. of magnesium in 20 ml. of tetrahydrofuran under nitrogen. After addition was complete, the dispersion was refluxed for 2 hours and then saccharin pseudo-chloride (3.5 g.) was added portionwise. An exotherm was observed, and the green solution that formed turned yellow-brown. The reaction solution was stirred overnight and then slowly added to 500 ml. of water. The precipitate that formed was slowly filtered and the solid obtained was dissolved in ether, dried and evaporated to yield 8.5 g. of light yellow solid having the formula:

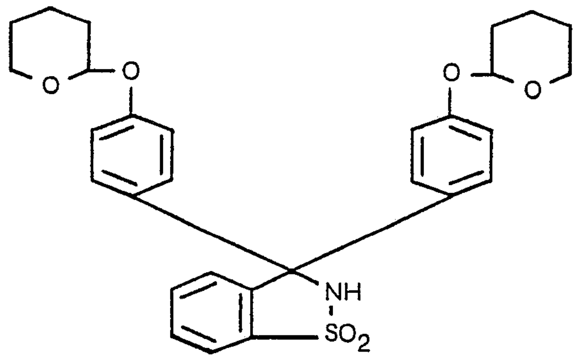

The compound prepared in step (a) was sequentially reacted with NaH and the appropriate acylating agents, namely,

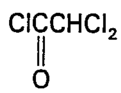 and 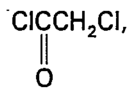

respectively, and then the protecting groups were removed by treating the N-acylated compounds with 10% HCl solution to yield the title compounds.

Example 29

Preparation of the compound having the formula:

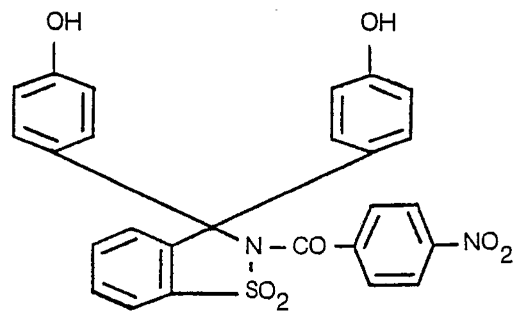

The title compound was prepared according to the procedure described in Examples 27 and 28 above except that the acylating agent employed was

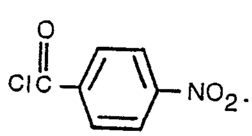

Examples 30—36

The compounds having the formulae:

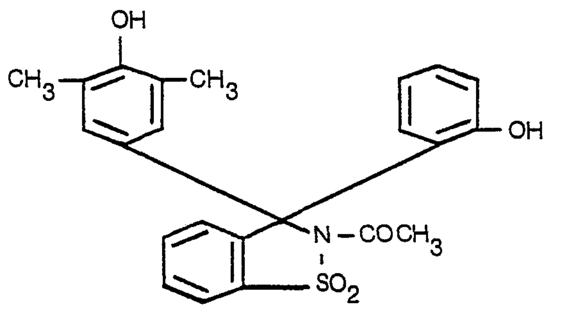

Example 30

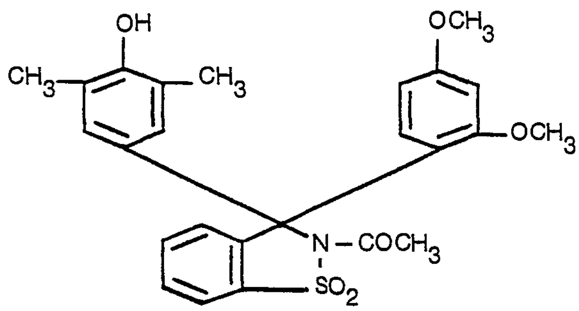

Example 31

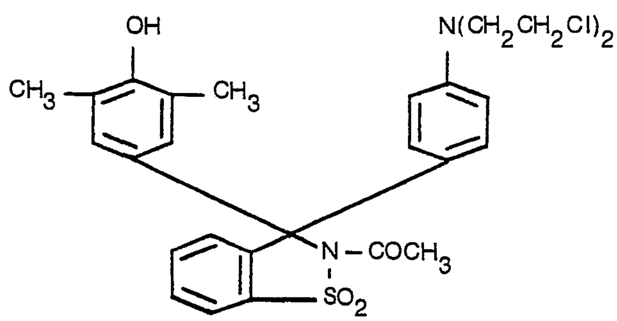

Example 32

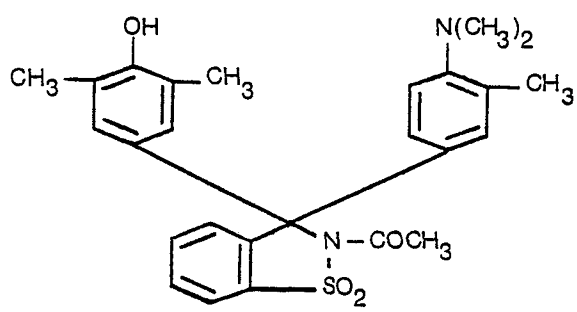

Example 33

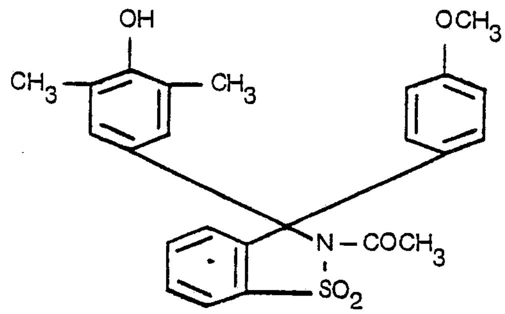

Example 34

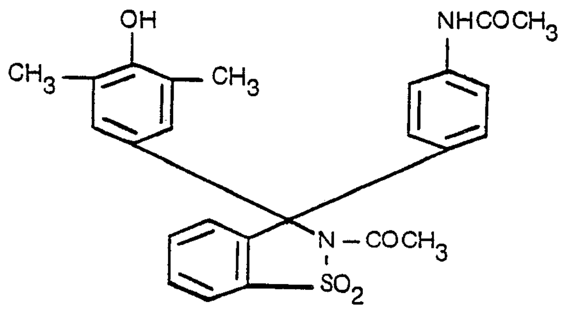

Example 35

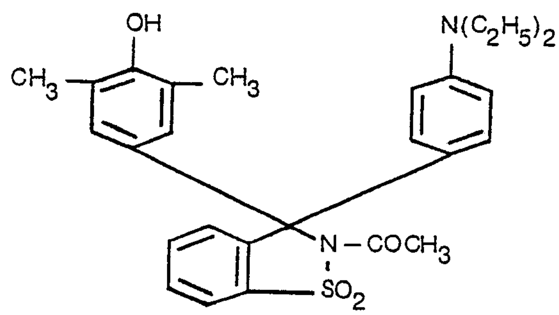

Example 36

were prepared by reacting 3-(3′,5′-dimethyl-4′-methoxymethoxy-1′-phenyl)benz[d]isothiazole-1,1-dioxide with the selected aryl lithium reagent as described in step (a) of Example 21 above to give the corresponding 3,3-disubstituted-2,3-dihydrobenz[d]isothiazole-1,1-dioxide. The lithium reagents employed were

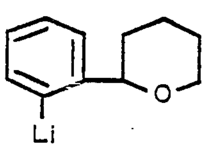 , 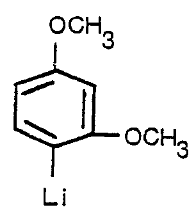 , 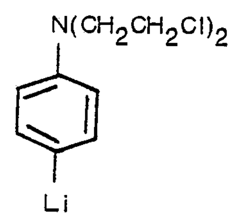

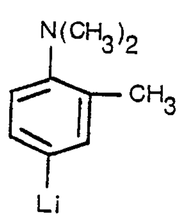 , 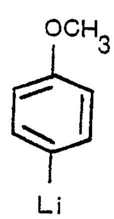 , 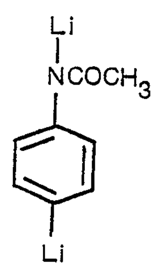

and 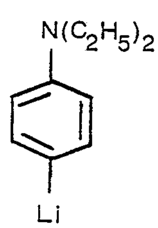 , respectively.

The 3,3 - disubstituted - 2,3 - dihydrobenz[d]isothiazole - 1,1 - dioxides obtained were then sequentially reacted with NaH and

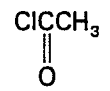

to give the corresponding

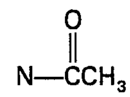

intermediates. The intermediates were then treated with dilute hydrochloric acid to remove the protecting group(s) to give the title compounds

Example 37

The compound having the formula

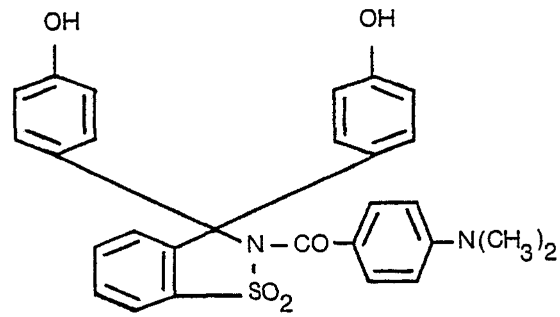

was prepared according to the procedure described in Examples 27 and 28 except that the acylating agent employed was

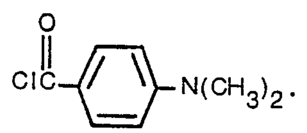

Example 38

Preparation of the compound having the formula:

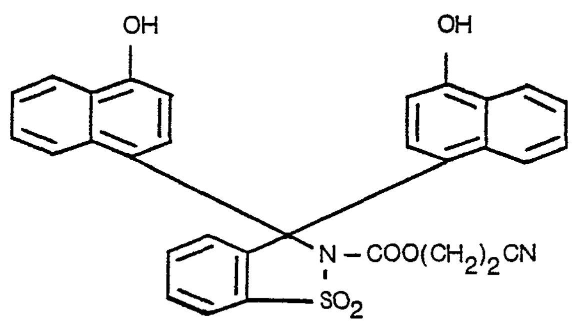

(a) To a solution of 168 mg. of t-potassium butoxide in 5 ml. of dry tetrahydrofuran was added 834 mg. of 3,3-di[4′-(2″-tetrahydropyranyloxy)-1′-naphthyl]-2,3-dihydrobenz[d] isothiazole-1,1-dioxide in several small portions under nitrogen. The yellow-brown solution obtained was stirred for 2 hours at room temperature and then cooled in an ice bath.

(b) To the cooled solution of step (a) was added dropwise 267 mg. of $\beta$-cyanoethylchloroformate. (The color changed to light yellow). The resulting solution was stirred over the weekend at room temperature under nitrogen during which time most of the solvent had evaporated leaving yellow-orange solids comprising the title compound and deblocked starting material.

(c) The yellow-orange solids were taken up in 25 ml. of ether and the ether solution concentrated to about 10 ml. The crystals that formed were filtered, washed with ether and the orange and yellow components separated by preparative TLC on (silica gel/ether). The orange component was removed by

eluting with n-butanol and the yellow component was removed by eluting with methanol. The methanol was then evaporated to give the title compound.

The intermediate having the formula

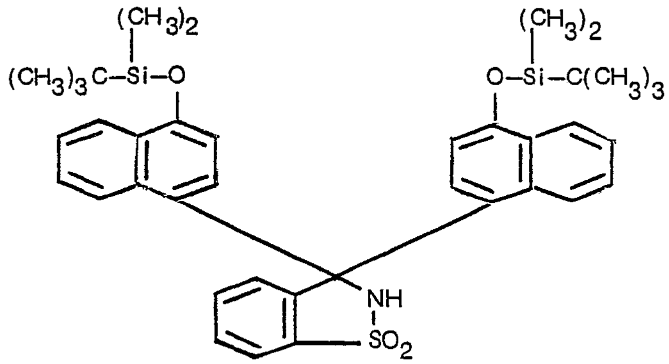

which is a compound according to the present invention was prepared as follows:

(a) 16.87 g. of 4-bromonaphthyl dimethyl-t-butylsilyl ether was dissolved in 60 ml. of anhydrous ethyl ether under nitrogen, and the solution then cooled to −60°C in a dry ice-acetone bath. 20.8 ml. of 2.4 N n-butyllithium in hexane was added to the cooled solution dropwise over a period of 15—20 minutes. The reaction mixture was stirred for 30 minutes and allowed to come to 15°C over 45 minutes.

(b) The solution of step (a) was then cooled back to −40°C, and 4.63 g. of saccharin pseudochloride was added portionwise. After addition was complete, the reaction mixture was allowed to warm to room temperature, stirred at room temperature overnight and cooled. 50 ml. of cold water was added followed by the addition of dilute HCl until the pH reached 3—4. The ether phase was separated, and the aqueous phase was extracted with 50 ml. of ether. The ether portions were combined, washed with 100 ml. of water, dried over sodium sulfate, filtered and evaporated under reduced pressure. The residue was dissolved in iso-propanol and the iso-propanol solution slowly poured into 1 liter aqueous HCl solution having a pH of 3.

The aqueous phase was decanted leaving a gummy residue. Treatment of the residue with iso-propanol and aqueous HCl (pH 3) was repeated and the final residue was dissolved in 250 ml. of iso-propanol. 3% HCl was added until precipitation ceased. The solution was allowed to stand for 10 min., the solvent decanted and water added to the gummy precipitate which on standing became crystalline. The precipitate was filtered and airdried over the weekend to give 15.55 g. of the title compound.

The dimethyl-t-butylsilyl ether of 4-bromo-1-naphthol was prepared as follows:

4-Bromo-1-naphthol (22.1 g.) and dimethyl-t-butylsilyl chloride (18.1 g.) were dissolved in 50 ml. of dimethylformamide at room temperature. The resulting solution was cooled in an ice bath and imidazole (17.0 g.) added under nitrogen. (A slight exotherm was observed). A solid precipitated and the reaction mixture was stirred overnight.

A small sample of the crude product was treated with water adjusted to a pH of about 4—5 with dilute HCl and the solids isolated and dried. TLC on silica gel using hexane showed the product but no starting material.

The reaction mixture remaining was poured into 1500 ml. of water at about 20°C with stirring. The pH was adjusted to 4—5 with dilute HCl, and the solids were filtered, washed with water, and air-dried for 2 hours and dissolved in 150 ml. of boiling isopropanol. The isopropanol solution was filtered while hot and then cooled slowly to room temperature. Crystals began to form and after standing at room temperature overnight, the solution was cooled in an ice water bath for 1 hour and filtered. The solid collected was washed with small amounts of isopropanol, air-dried briefly and then dried in vacuo for 2 hours to give 24.3 g. of the title compound (melting range 70°—73°C).

The 3,3-di[4′-(2″-tetrahydropyranyloxy)-1′-phenyl]-2,3-dihydrobenz[d]isothiazole-1,1-dioxide having the formula

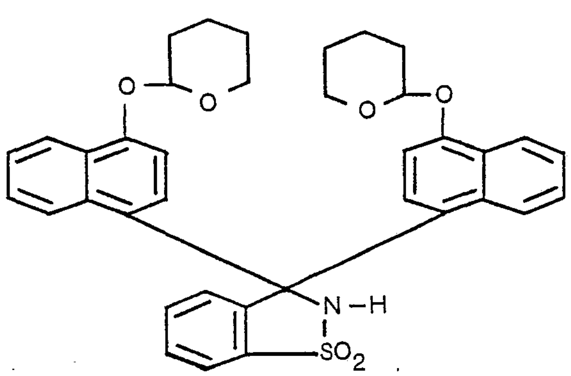

was prepared in the same manner as the phenol analog described in part (a) of Examples 15, 16, 18, 27 and 28. The methoxymethyl ether and 2′-tetrahydropyranyl ether of 4-bromo-1-naphthol were prepared according to the procedures described above and the methoxymethyl and 2′-tetrahydropyranyl ethers of 4-bromo-1-naphthol were converted to the corresponding 4-lithium derivatives by reaction with n-butyllithium according to the procedures described above.

Typical of the sulfam(na)phthaleins that may be prepared from the novel compounds of the present invention are those represented by the following formula:

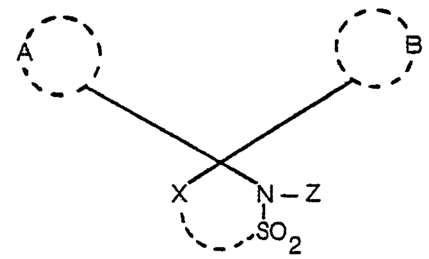

wherein

A is a 4′-hydroxy-1′-phenyl moiety, unsubstituted or substituted with one or more substituents compatible with organometallic reagents, the same or different, or a 4′-hydroxy-1′-naphthyl moiety, unsubstituted or substituted with one or more substituents compatible with organometallic reagents, the same or different; B is a phenyl moiety, unsubstituted or substituted with one or more substituents compatible with organometallic reagents, the same or different, or a naphthyl moiety, unsubstituted or substituted with one or more substituents compatible with organometallic reagents, the same or different; X represents the carbon atoms necessary to complete a 2,3-dihydrobenz[d]isothiazole-1,1-dioxide moiety or a 2,3-dihydronaphtho[1,8-de]-1,2-thiazine-1,2-dioxide moiety; and Z represents a carbonyl moiety containing a

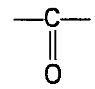

group bonded to said N atom.

Typical substituents compatible with or capable of being protected to be compatible with organometallic reagents include branched or straight chain alkyl, such as, methyl, ethyl, isopropyl, n-butyl, t-butyl, hexyl, octyl, dodecyl, hexadecyl, octadecyl and eicosanyl; aryl, such as phenyl and naphthyl; alkaryl and aralkyl, preferably, alkyl-substituted phenyl and phenyl-substituted alkyl, such as p-ethylphenyl, p-octylphenyl, p-dodecylphenyl, benzyl, phenylhexyl and phenyldodecyl; alkoxy, such as, methoxy, ethoxy, butoxy, octadecyloxy, 1-ethoxy-2-(β-ethoxyethoxy); aryloxy, such as, phenoxy, benzyloxy and naphthoxy; alkoxyalkyl, such as, methoxymethyl, ethoxymethyl, and dodecyloxyethyl; halo, such as fluoro, bromo and chloro; trihalomethyl, such as trifluoromethyl and trichloromethyl; sulfonamido (—NH—$SO_2R°$ wherein R° is alkyl, aryl, alkaryl or aralkyl); sulfamoyl (—$SO_2$—NH—R° wherein R° has the same meaning given above); acyl

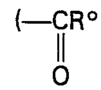

wherein

R° has the meaning given above); sulfanyl (—$SO_2$—R° wherein R° has the same meaning given above); sulfo; cyano, carboxy, hydroxy; and amino including mono- and disubstituted amino (—NR′R″ wherein R′ and R″ each are hydrogen, alkyl, aryl, alkaryl or aralkyl or R′ and R″ taken together represent the atoms necessary to complete a saturated heterocyclic ring, such as piperidino, pyrrolidino, N-lower alkylpiperazino, morpholino, thiomorpholino and tetrahydro-2H,4H-1,3,6-dioxazocino or a fused heterocyclic ring system, e.g., quinolizidine).

In a preferred embodiment of the present invention, the method of synthesizing N-substituted sulfam(na)phthaleins comprises (1a) reacting (a) a compound of the formula

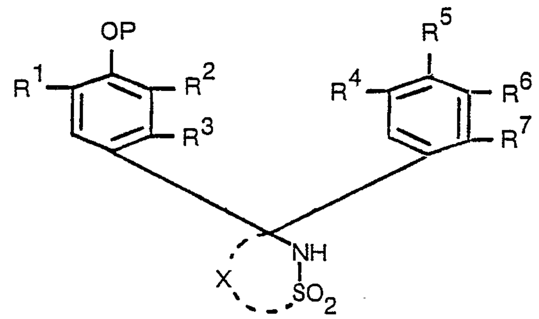

wherein

P is a protecting group selected from 2′-tetrahydropyranyl, methoxymethyl and dimethyl-t-butylsilyl; $R^1$ and $R^2$ are selected from hydrogen, alkyl, alkoxy, chloro and fluoro; $R^3$ is hydrogen, alkyl, alkoxy, or —OP; $R^2$ and $R^3$ taken together represent the carbon atoms necessary to complete a fused benzene ring; $R^4$ and $R^6$ each are selected from hydrogen, alkyl, alkoxy, chloro and fluoro; $R^5$ is hydrogen, alkyl, alkoxy, —OP′ wherein P′ has the same meaning as P, —N,N-(dialkyl)-amino, —N,N-(ω—$R^8$ alkyl)$_2$-amino wherein $R^8$ is halo or —OP″ wherein P″ has the same meaning as P, —NHCOCH$_3$, piperidino, pyrrolidino, N-methylpiperazino, morpholino, thiomorpholino or tetrahydro-2H,4H-1,3,6-dioxazocino; $R^7$ is hydrogen, alkyl, alkoxy or —OP‴ wherein P‴ has the same meaning as P; $R^6$ and $R^7$ taken together represent the carbon atoms necessary to complete a fused benzene ring; $R^4$, $R^5$ and $R^6$ taken together represent the atoms necessary to complete a fused [ij] quinolizidine ring; and X represents the atoms necessary to complete 2,3-dihydrobenz[d]isothiazole-1,1-dioxide or 2,3-dihydronaphtho[1,8-de]-1,2-thiazine-1,1-dioxide with (b) an acid halide of the formula

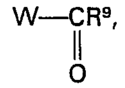

wherein

W is chloro or bromo and wherein $R^9$ is selected from methyl, methyl substituted with at least one halogen group selected from chloro, bromo and fluoro, alkoxy having 1 to 4 carbon atoms, phenyl, phenyl substituted in the para position with alkyl having 1 to 4 carbon atoms or —N,N-(dialkyl)-amino, phenyl substituted with at least one electron-withdrawing group, phenoxy, phenoxy substituted with at least one electron-withdrawing group, —O(CH$_2$)$_2$Y wherein Y is an electron-withdrawing group and phenyl substituted in the ortho position with —CH$_2$R$^{10}$ wherein $R^{10}$ is chloro or bromo in pyridine at a temperature between about 0° and 100°C to yield (c) the corresponding N-acylated compound of the formula:

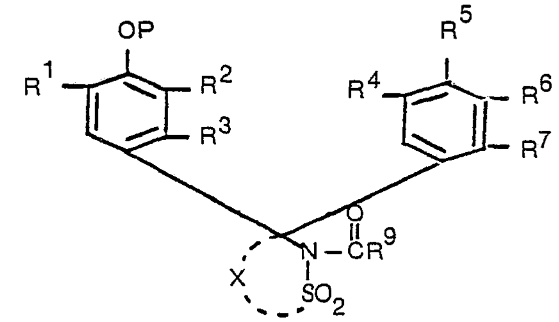

wherein

P, $R^1$, $R^2$, $R^3$, $R^4$, $R^5$, $R^6$, $R^7$, $R^9$ and X have the same meaning given above; or (1b) sequentially reacting said compound (a) with MH wherein M is an alkali metal selected from lithium, sodium and potassium in an inert organic solvent at a temperature between about 0° and 100°C to give the corresponding N-alkali metal salt having the formula

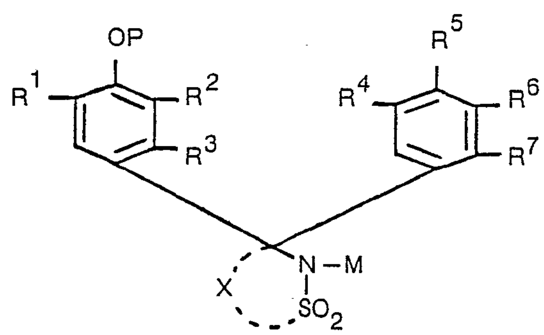

wherein

P, $R^1$, $R^2$, $R^3$, $R^4$, $R^5$, $R^6$, $R^7$, X and M have the same meaning given above and then reacting said N-alkali metal salt with said acid halide (b) to give the said N-acylated compound (c); and

(2) treating said compound (c) between about 20°C and 100°C with an organic or inorganic acid at a pH between about 0.1 and 5 to yield the product having the formula

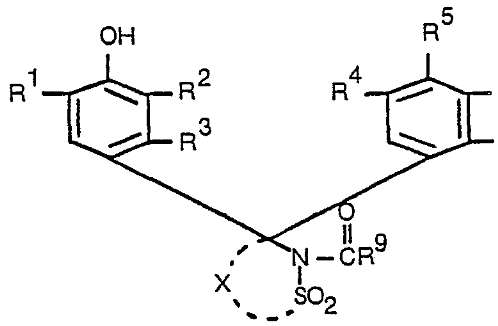

wherein

$R^1$, $R^2$, $R^3$, $R^4$, $R^5$, $R^6$, $R^7$, $R^9$ and X have the same meaning given above except that said —OP of $R^3$, said —$OP^I$ and —$OP^{II}$ of $R^5$, and said —$OP^{III}$ of $R^7$ each are —OH. By electron-withdrawing group is intended "a group with a positive sigma value as defined by Hammett's Equation".

Usually, the alkyl and the alkoxy substitutents comprising $R^1$, $R^2$, $R^3$, $R^4$, $R^5$, $R^6$ and $R^7$ are lower alkyl having 1 to 4 carbon atoms, such as, methyl, ethyl, n-propyl, isopropyl and n-butyl and lower alkoxy having 1 to 4 carbon atoms, such as, methoxy, ethoxy, propoxy and butoxy. Also the alkyl groups of the —N,N-(dialkyl)amino and —N,N-(ω—$R^8$alkyl)$_2$-amino substituents usually are lower alkyl groups include fluoro, chloro and bromo; nitro; cyano;

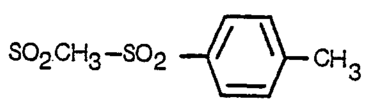 and $-COCH_3$.

The value for these and other groups have been reported by Eugen Müller, Methoden Der Organischen Chemie, Georg Thieme Verlag, Stuttgart, 1970, p. 78.

In a particularly preferred embodiment, X represents the atoms necessary to complete 2,3-dihydrobenz[d]isothiazole-1,1-dioxide.

Specific examples of compounds that may be prepared from the novel compounds of the present invention are as follows:

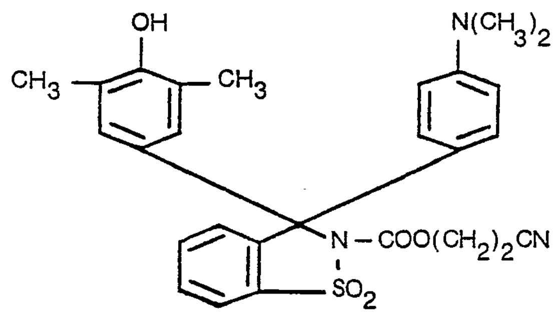

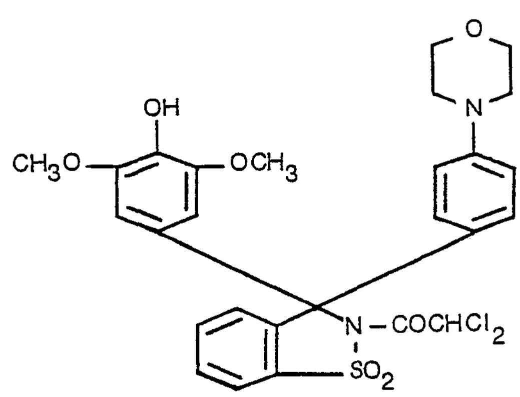

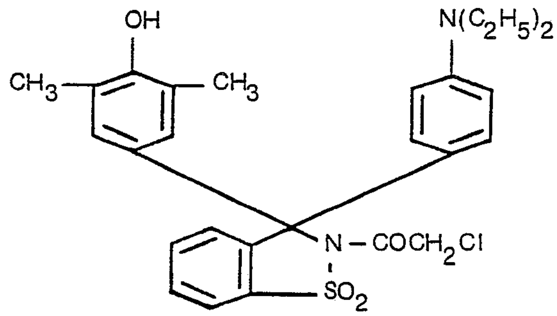

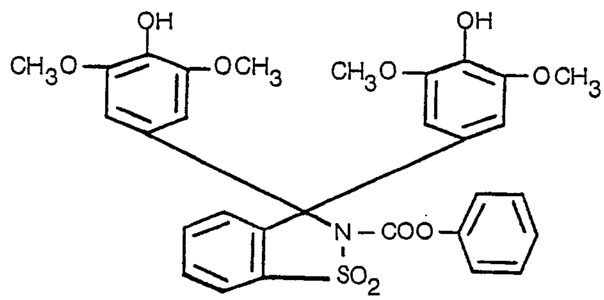

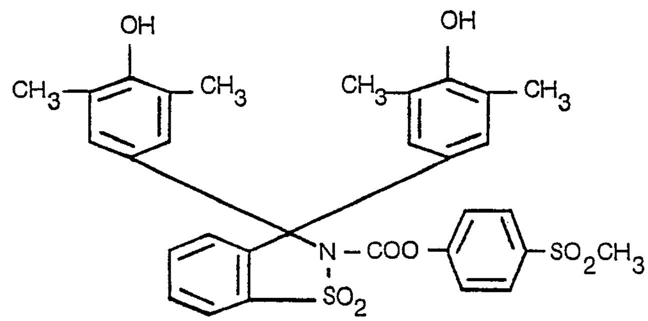

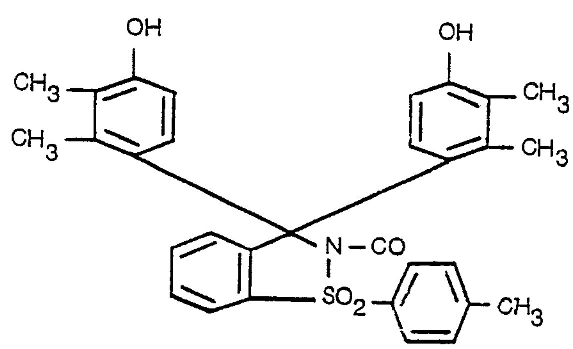

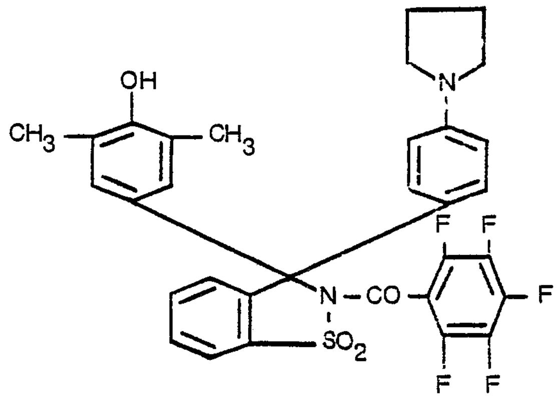

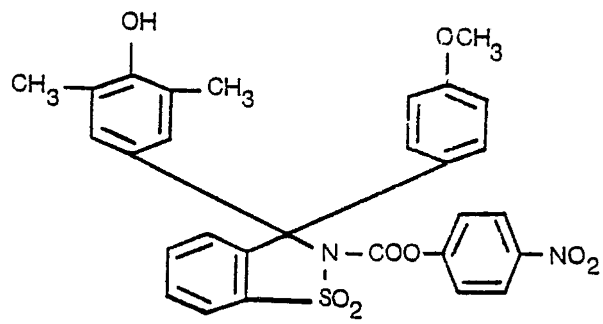

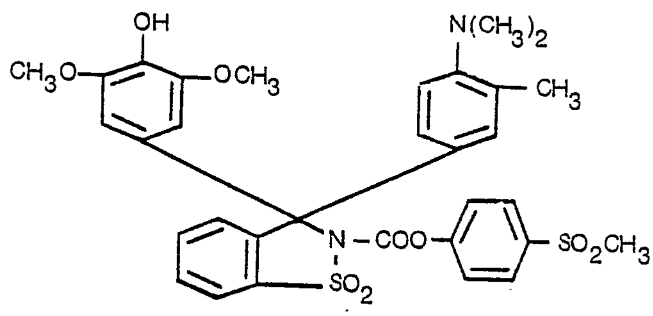

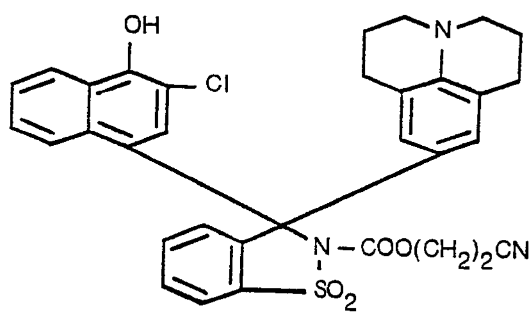

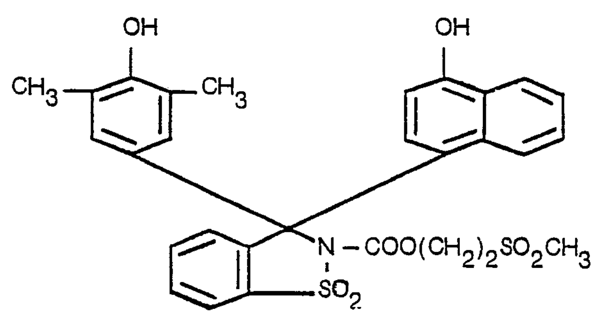

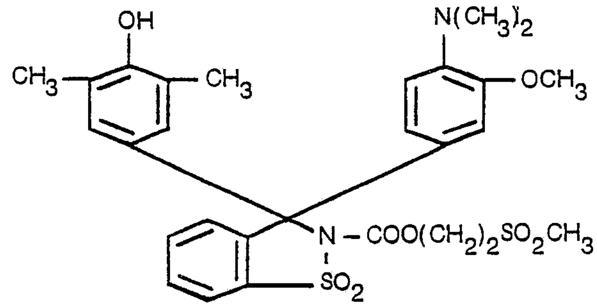

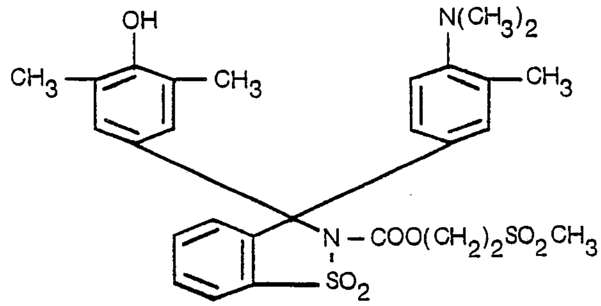

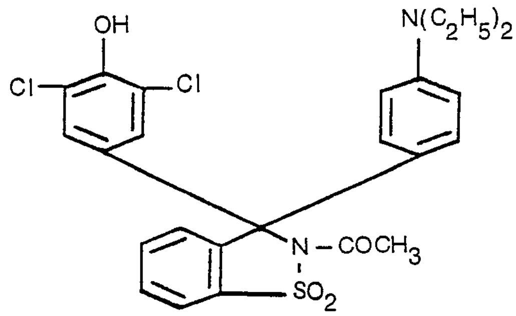

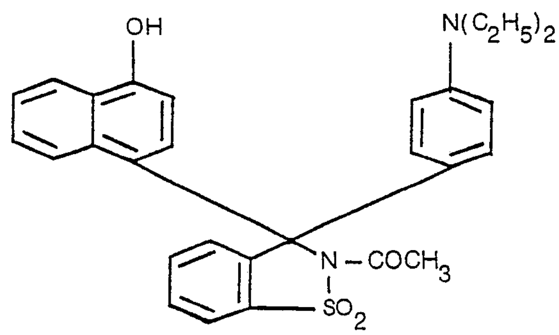

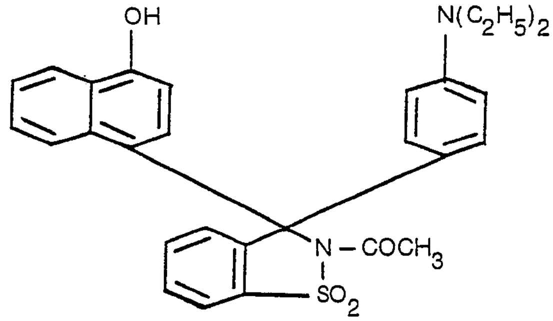

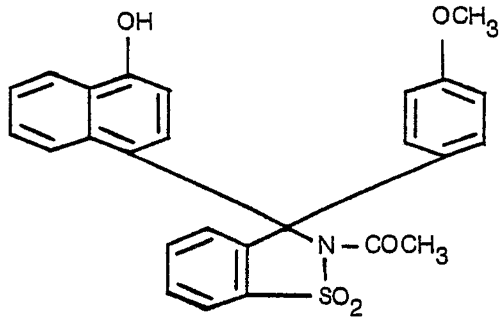

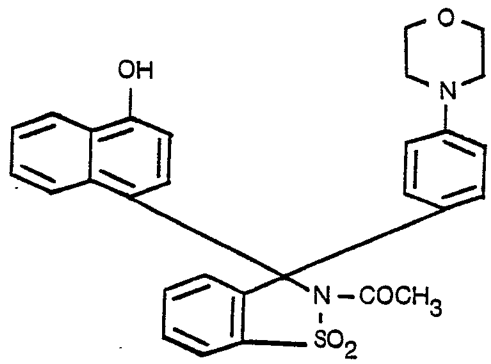

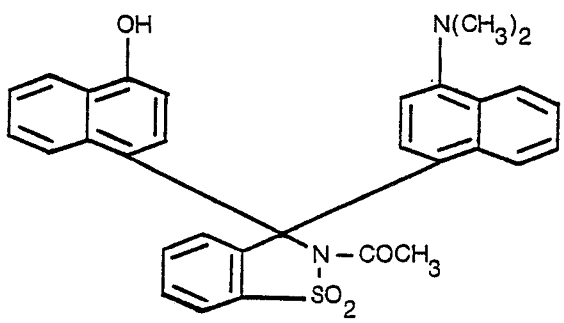

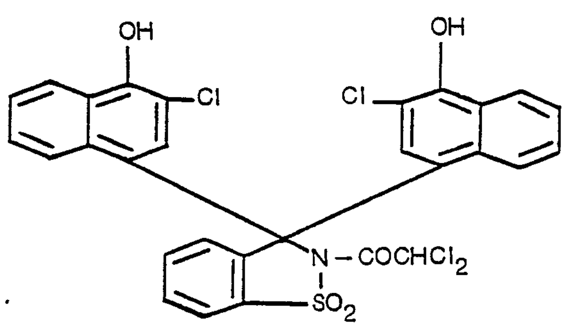

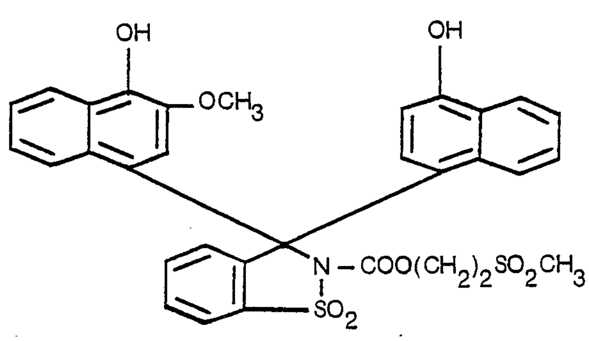

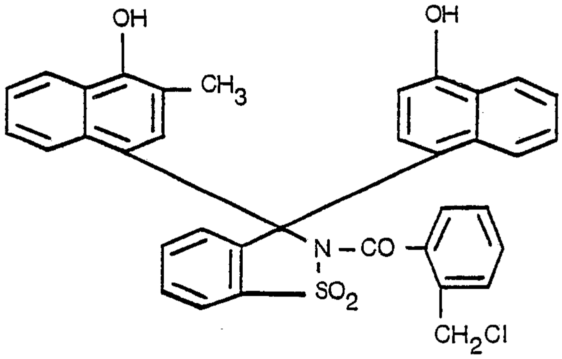

## Claims

1. A compound of the formula

(I) 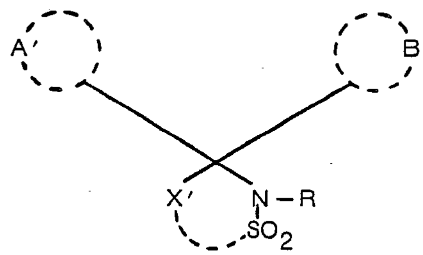

wherein

A′ is a 4′-OP-1′-phenyl moiety, unsubstituted or substituted with one or more substituents compatible with organometallic reagents, the same or different, or a 4′-OP-1′-naphthyl moiety, unsubstituted or substituted with one or more substituents compatible with organometallic reagents, the same or different; B is a phenyl moiety, unsubstituted or substituted with one or more substituents compatible with organometallic reagents, the same or different, or a naphthyl moiety, unsubstituted or substituted with one or more substituents compatible with organometallic reagents, the same or different; P is a protecting group selected from 2′-tetrahydropyranyl, methoxymethyl and dimethyl-t-butyl-silyl; X′ represents the atoms necessary to complete a 2,3-dihydrobenz[d]isothiazole-1,1-dioxide moiety or a 2,3-dihydronaphtho[1,8-de]-1,2-thiazine-1,1-dioxide moiety; and R is hydrogen, an alkali metal selected from sodium, potassium and lithium or a carbonyl moiety wherein the

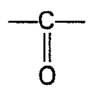

is bonded directly to said N atom.

2. A compound as defined in claim 1 and having the formula

(II) 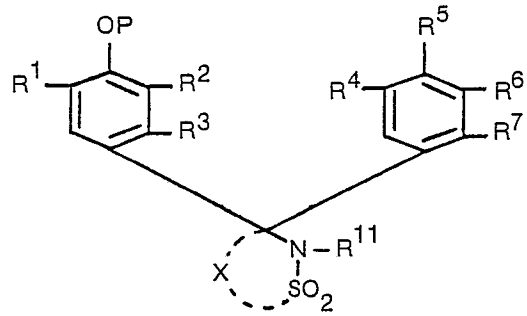

wherein

P is a protecting group selected from 2′-tetrahydropyranyl, methoxymethyl and dimethyl-t-butyl-silyl; $R^1$ and $R^2$ are selected from hydrogen, alkyl, alkoxy, chloro and fluoro; $R^3$ is hydrogen, alkyl, alkoxy, or —OP; or $R^2$ and $R^3$ taken together represent the carbon atoms necessary to complete a fused benzene ring; $R^4$ and $R^6$ each are selected from hydrogen, alkyl, alkoxy, chloro and fluoro; $R^5$ is hydrogen, alkyl, alkoxy, —OP' wherein P' has the same meaning as P, —N,N-(dialkyl)-amino, —N,N-

($\omega$—$R^8$ alkyl)$_2$-amino wherein $R^8$ is halo or —$OP^{II}$ wherein $P^{II}$ has the meaning as P, —$NHCOCH_3$, piperidino, pyrrolidino, N-methylpiperazino, morpholino, thiomorpholino or tetrahydro-2H,4H-1,3,6-dioxazocino; $R^7$ is hydrogen, alkyl, alkoxy or —$OP^{III}$ wherein $P^{III}$ has the same meaning as P; or $R^6$ and $R^7$ taken together represent the carbon atoms necessary to complete a fused benzene ring; or $R^4$, $R^5$ and $R^6$ taken together represent the atoms necessary to complete a fused [ij] quinolizidine ring; X represents the atoms necessary to complete 2,3-dihydrobenz[d]isothiazole-1,1-dioxide or 2,3-dihydronaphtho[1,8-de]-1,2-thiazine-1,1-dioxide and $R^{11}$ is hydrogen, an alkali metal selected from sodium, potassium and lithium or

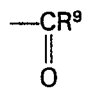

wherein

$R^9$ is selected from methyl, methyl substituted with at least one halogen atom selected from chloro, bromo and fluoro, alkoxy having 1 to 4 carbon atoms, phenyl, phenyl substituted in the para position with alkyl having 1 to 4 carbon atoms or —N,N-(dialkyl)-amino, phenyl substituted with at least one electron-withdrawing group, phenoxy, phenoxy substituted with at least one electron-withdrawing group, —$O(CH_2)_2Y$ wherein Y is an electron-withdrawing group and phenyl substituted in the ortho position with —$CH_2R^{10}$ wherein $R^{10}$ is chloro or bromo.

3. A compound as defined in claim 2, wherein said $R^1$, $R^2$, $R^4$ and $R^6$ each are methyl, OP and $R^5$ are methoxymethyl, $R^3$, $R^7$ and $R^{11}$ are hydrogen, and X represents the atoms necessary to complete a 2,3-dihydrobenz[d]isothiazole-1,1-dioxide structure.

4. A compound as defined in claim 2 wherein said $R^1$ and $R^2$ are methyl; $R^3$, $R^4$, $R^6$ and $R^7$ are hydrogen; and $R^5$ is morpholino; —N,N-(dialkyl)amino; —N,N-($\omega$—$R^8$-alkyl)$_2$-amino; alkoxy; —$NHCOCH_3$; pyrrolidino; or tetrahydro-2H, 4H-1,3,6-dioxazocino.

5. A compound as defined in claim 2 wherein said $R^1$ and $R^2$ are methoxy, $R^3$, $R^4$ and $R^6$ are hydrogen and $R^5$ and $R^7$ are methoxy.

## Revendications

1. Composé de formule

(I) 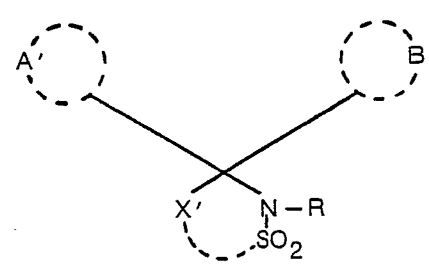

où A′ est un fragment 4′—OP—1′-phényle, non-substitué ou substitué par un ou plusieurs substituants compatibles avec des réactifs organo-métalliques, identiques ou différents, ou un fragment 4′—OP—1′-naphtyle, non-substitué ou substitué par un ou plusieurs substituants compatibles avec des réactifs organo-métalliques, identiques ou différents; B est un fragment phényle, non-substitué ou substitué par un ou plusieurs substituants compatibles avec des réactifs organo-métalliques, identiques ou différents, ou un fragment naphtyle, non-substitué ou substitué par un ou plusieurs substituants compatibles avec des réactifs organo-métalliques, identiques ou différents; P est un groupe protecteur choisi entre les radicaux 2′-tétrahydropyranyle, méthoxyméthyle et diméthyl-tert-butyl-silyle; X′ représente les atomes nécessaires pour compléter un fragment 2,3-dihydrobenzo[d]isothiazole-1,1-dioxyde ou un fragment 2,3-dihydronaphto[1,8-de]-1,2-thiazine-1,1-dioxyde; et R est l'hydrogène, un métal alcalin choisi entre le sodium, le potassium et le lithium, ou un fragment carbonyle dans lequel le

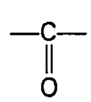

est directement fixé audit atome N.

2. Composé selon la revendication 1 et ayant la formule

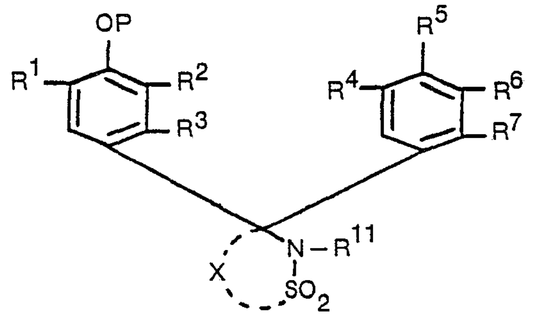

où P est un groupe protecteur choisi parmi les radicaux 2'-tétrahydropyranyle, méthoxyméthyle et diméthyl-tert-butyl-silyle; $R^1$ et $R^2$ sont choisis entre l'hydrogène, les radicaux alkyle, alcoxy, chloro et fluoro; $R^3$ est l'hydogène ou un radical alkyle, alcoxy ou —OP; ou bien $R^2$ et $R^3$, pris ensemble représentent les atomes de carbone nécessaires pour compléter un noyau benzénique condensé; $R^4$ et $R^6$ sont chacun choisis entre l'hydrogène ou les radicaux alkyle, alcoxy, chloro et fluoro; $R^5$ est l'hydrogène, un radical alkyle, alcoxy, —$OP^I$ où $P^I$ a la même signification que P, —N,N-(dialkyl)-amino, —N,N-($\omega$—$R^8$alkyl)$_2$-amino où $R^8$ est un radical halogéno, ou —$OP^{II}$ où $P^{II}$ a la même signification que P, —$NHCOCH_3$, pipéridino, pyrrolidino, N-méthylpipérazino, morpholino, thiomorpholino ou tétrahydro-2H,4H-1,3,6-dioxazocino; $R^7$ est l'hydrogène ou un radical alkyle, alcoxy ou —$OP^{III}$ où $P^{III}$ a la même signification que P; ou bien $R^6$ et $R^7$, pris ensemble, représentent les atomes de carbone nécessaires pour compléter un noyau de benzène condensé; ou bien $R^4$, $R^5$ et $R^6$ pris ensemble représentent les atomes nécessaires pour compléter un noyau [ij]quinolizidine condensé; X représente les atomes nécessaires pour compléter le 2,3-dihydrobenzo[d]isothiazole-1,1-dioxyde ou le 2,3-dihydronaphto[1,8-de]-1,2-thiazine-1,1-dioxyde, et $R^{11}$ est l'hydrogène, un métal alcalin choisi entre le sodium, le potassium et le lithium, ou

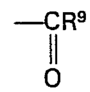

où $R^9$ est choisi parmi les radicaux méthyle, méthyle substitué par au moins un atome d'halogène choisi entre le chlore, le brome et le fluor, alcoxy à 1 à 4 atomes de carbone, phényle, phényle substitué en position para par un radical alkyle à 1 à 4 atomes de carbone ou —N,N-(dialkyl)-amino, phényle substitué par au moins un groupe attracteur d'électrons, phénoxy, phénoxy substitué par au moins un groupe attracteur d'électrons, —$O(CH_2)_2Y$ où Y est un groupe attracteur d'électrons et phényle substitué en position ortho par —$CH_2R^{10}$ où $R^{10}$ est le radical chloro ou bromo.

3. Composé selon la revendication 2, caractérisé en ce que lesdits $R^1$, $R^2$, $R^4$ et $R^6$ sont des radicaux méthyle, OP et $R^5$ sont des radicaux méthoxyméthyle, $R^3$, $R^4$ et $R^{11}$ sont l'hydrogène, et X représente les atomes nécessaires pour compléter une structure de 2,3-dihydrobenzo[d]isothiazole-1,1-dioxyde.

4. Composé selon la revendication 2, caractérisé en ce que lesdits $R^1$ et $R^2$ sont des radicaux méthyle; $R^3$, $R^4$, $R^6$ et $R^7$ sont l'hydrogène; et $R^5$ est le radical morpholino; —N,N-(dialkyl)-amino; —N,N-($\omega$—$R^8$-alkyl)$_2$-amino; alcoxy; —$NHCOCH_3$; pyrrolidino; ou tétrahydro-2H,4H-1,3,6-dioxazocino.

5. Composé selon la revendication 2, caractérisé en ce que lesdits $R^1$ et $R^2$ sont des radicaux méthoxy, $R^3$, $R^4$ et $R^6$ sont l'hydrogène, et $R^5$ et $R^7$ sont des radicaux méthoxy.

**Patentansprüche**

1. Verbindung der Formel

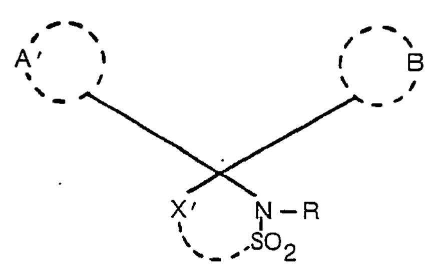

worin bedeuten; A' bedeutet unsubstituierte oder mit einem oder mehreren Substituenten, die mit Organometallreagentien verträglich sind, substituiert und gleich oder voneinander verschieden sind, oder unsubstituierte oder mit einem oder mehreren Substituenten, die mit Organometallreagentien verträglich sind, substituiert und gleich oder voneinander verschieden sind; B bedeutet unsubstituierte

oder mit einem oder mehreren Substituenten, die mit Organometallreagentien verträglich sind, substituiert und gleich oder voneinander verschieden sind, oder unsubstituierte oder mit einem oder mehreren Substituenten, die mit Organometallreagentien verträglich sind, substituiert und gleich oder voneinander verschieden sind; P bedeutet eine Schutzgruppe, ausgewählt aus den Gruppen 2′-Tetrahydropyranyl, Methoxymethyl und Dimethyl-t-butylsilyl; X′ bedeutet die zur Vervollständigung einer 2,3-Dihydrobenz[d]isothiazol-1,1-dioxidgruppe oder einer 2,3-Dihydronaphtho[1,8-de]-1,2-thiazin-1,1-dioxydgruppe erforderlichen Atome; und R bedeutet Wasserstoff, ein Alkalimetall, ausgewählt aus der Gruppe Natrium, Kalium und Lithium, oder eine Carbonylgruppe, worin die Gruppe

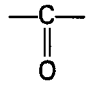

direkt an das N-Atom gebunden ist.

2. Verbindung nach Anspruch 1, mit der Formel

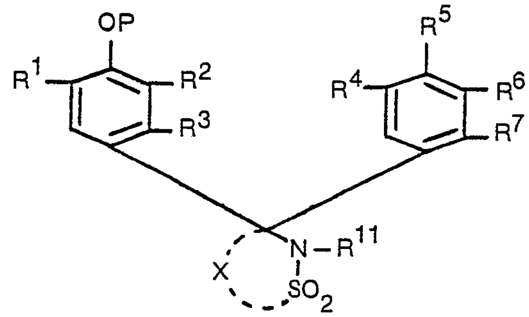

worin bedeuten: P ist eine Schutzgruppe, ausgewählt aus den Gruppen 2′-Tetrahydropyranyl, Methoxymethyl und Dimethyl-t-butylsilyl; $R^1$ und $R^2$ sind ausgewählt aus Wasserstoff, Alkyl, Alkoxy, Chlor und Fluor; $R^3$ bedeutet Wasserstoff, Alkyl, Alkoxy oder —OP; oder $R^2$ und $R^3$, zusammengenommen, bedeuten die Kohlenstoffatome, die zur Vervollständigung eines kondensierten Benzolringes erforderlich sind; $R^4$ und $R^6$ bedeuten jeweils Wasserstoff, Alkyl, Alkoxy, Chlor bzw. Fluor; $R^5$ bedeutet Wasserstoff, Alkyl, Alkoxy, —OP′ (worin P′ die gleiche Bedeutung wie P hat), —N,N-(Dialkyl)-amino, —N,N-($\omega$—$R^8$alkyl)$_2$-amino (worin $R^8$ Halogen oder —OP″ bedeutet, worin P″ die gleiche Bedeutung hat wie P), —$NHCOCH_3$, Piperidino, Pyrrolidino, N-Methylpiperazino, Morpholino, Thiomorpholino oder Tetrahydro-2H,4H-1,3,6-dioxazozino; $R^7$ bedeutet Wasserstoff, Alkyl, Alkoxy oder —OP‴ (worin P‴ die gleiche Bedeutung wie P hat); oder $R^6$ und $R^7$, zusammengenommen, bedeuten die zur Vervollständigung eines kondensierten Benzolrings erforderlichen Atome; oder $R^4$, $R^5$ und $R^6$, zusammengenommen, bedeuten die zur Vervollständigung eines kondensierten [ij]-Chinolizidinringes erforderlichen Atome; X bedeutet die zur Vervollständigung von 2,3-Dihydrobenz[d]isothiazol-1,1-dioxid oder 2,3-Dihydronaphtho[1,8-de]-1,2-thiazin-1,1-dioxid erforderlichen Atome, und $R^{11}$ bedeutet Wasserstoff, ein Alkalimetall aus der Gruppe Natrium, Kalium bzw. Silicium oder

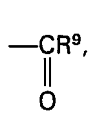

worin $R^9$ eine Methylgruppe, eine mit mindestens einem Halogenatom (ausgewählt aus Chlor, Brom bzw. Fluor) eine Alkoxygruppe mit 1 bis 4 Kohlenstoffatomen, eine Phenylgruppe, eine in der para-Stellung mit einer Alkylgruppe mit 1 bis 4 Kohlenstoffatomen oder mit —N,N-(Dialkyl)-amino substituierte Phenylgruppe, eine mit mindestens einer elektronenentziehenden Gruppe substituierte Phenylgruppe, eine Phenoxygruppe, eine mit mindestens einer elektronenentziehenden Gruppe substituierte Phenoxygruppe, die Gruppe —$O(CH_2)_2Y$ (worin Y eine elektronenentziehende Gruppe bedeutet), eine substituierte Phenoxygruppe bzw. eine Phenylgruppe bedeutet, die in ortho-Stellung mit —$CH_2R^{10}$ substituiert ist, worin $R^{10}$ Chlor oder Brom bedeutet.

3. Verbindung nach Anspruch 2, worin bedeuten: $R^2$, $R^4$ und $R^6$ bedeuten jeweils Methyl, OP und $R^5$ bedeuten Methoxymethyl, $R^3$, $R^4$ und $R^{11}$ bedeuten Wasserstoff, und X bedeutet die zur Vervollständigung einer 2,3-Dihydrobenz[d]isothiazol-1,1-dioxid-Struktur erforderlichen Atome.

4. Verbindung nach Anspruch 2, worin bedeuten: $R^1$ und $R^2$ bedeuten Methyl; $R^3$, $R^4$, $R^6$ und $R^7$ bedeuten Wasserstoff; und $R^5$ bedeutet eine der Gruppen Morpholino; N,N-(Dialkyl)-amino; N,N-($\omega$—$R^8$-Alkyl)$_2$-amino; Alkoxy; —$NHCOCH_3$; Pyrrolidino; oder Tetrahydro-2H,4H-1,3,6-dioxyzocino.

5. Verbindung nach Anspruch 2, worin $R^1$ und $R^2$ Methoxy, $R^3$, $R^4$ und $R^6$ Wasserstoff und $R^5$ und $R^7$ Methoxy bedeuten.